# EUROPEAN PATENT APPLICATION

(11) **EP 4 471 055 A1**
(43) Date of publication of application: **04.12.2024**
(21) Application number: 23746324.5
(22) Date of filing: 20.01.2023
(51) Int. Cl.: C07K 16/08, A61K 39/12, A61K 39/42, A61K 39/29, C07K 14/02, C07K 7/08, A61K 49/16, A61K 51/10, C12P 21/08, C12N 15/63, G01N 33/576

(54) **EPITOPE PEPTIDE AND ANTIBODY FOR TREATING HBV INFECTION AND RELATED DISEASES**

(30) Priority: 25.01.2022 CN 202210085365
(71) Applicant: Xiamen University, Xiamen, Fujian 361005 (CN); Yang Sheng Tang Company, Ltd., Hangzhou, Zhejiang 310000 (CN)
(72) Inventor: LUO, Wenxin, Xiamen, Fujian 361005 (CN); WANG, Yue, Xiamen, Fujian 361005 (CN); MEI, Yaxian, Xiamen, Fujian 361005 (CN); AO, Zhenghong, Xiamen, Fujian 361005 (CN); CHEN, Yuanzhi, Xiamen, Fujian 361005 (CN); TANG, Jixian, Xiamen, Fujian 361005 (CN); JIANG, Yichao, Xiamen, Fujian 361005 (CN); CHEN, Xiaoqing, Xiamen, Fujian 361005 (CN); ZHANG, Tianying, Xiamen, Fujian 361005 (CN); YUAN, Quan, Xiamen, Fujian 361005 (CN); XIA, Ningshao, Xiamen, Fujian 361005 (CN)
(74) Representative: Meissner Bolte Partnerschaft mbB
(86) International application number: PCT/CN2023/073343
(87) International publication number: WO 2023/143445

(57) **Abstract**

The present invention relates to the field of molecular virology and immunology, in particular to the field of hepatitis B virus (HBV) infection treatment. In particular, the present invention relates to an epitope peptide (or a variant thereof) capable of treating a hepatitis B virus infection, a recombinant protein comprising the epitope peptide (or a variant thereof) and a carrier protein, and an antibody (e.g. a nanobody) for the epitope peptide. The epitope peptide and antibody of the present invention can be used to prevent and/or treat HBV infection or a disease related to HBV infection (e.g. hepatitis B), for reducing the serum level of HBV DNA and/or HBsAg in a subject (e.g. a human), or for activating a subject (e.g. a chronic HBV infected person or a chronic hepatitis B patient) against HBV humoral immune response.

## Description

### Technical Field

The present invention relates to the fields of molecular virology and immunology, especially the field of treatment of hepatitis B virus (HBV) infection. Specifically, the present invention relates to an epitope peptide (or variant thereof) that can be used to treat hepatitis B virus infection, a recombinant protein comprising such epitope peptide (or variant thereof) and carrier protein, and an antibody (e.g., nanobody) targeting such epitope peptide. The epitope peptide and antibody of the present invention can be used to prevent and/or treat an HBV infection or a disease related to HBV infection (e.g., hepatitis B), to neutralize the virulence of HBV in a subject (e.g., a human), and to reduce the serum levels of HBV DNA and/or HBsAg in a subject, or be used to activate an humoral immune response against HBV in a subject (e.g., a chronic HBV infected person or a chronic hepatitis B patient).

### Background Art

HBV is a DNA virus that can cause a series of liver diseases of varying degrees, wherein chronic hepatitis B (CHB) infection has a huge risk of developing severe liver diseases, including liver cirrhosis (LC) and primary hepatocellular carcinoma (HCC). HBV is a serious public health burden worldwide.

The ideal treatment endpoint for CHB patients is to achieve HBsAg serological negative conversion (HBsAg loss). However, due to the high titer of HBsAg in patients, they develop immune tolerance, and the therapy based on existing drugs (interferon or nucleoside/nucleotide analogues) is difficult to achieve the desired end point. Therefore, it is urgent and necessary to develop innovative therapeutic drugs and methods for patients with chronic HBV infection.

The development of new drugs to treat chronic HBV infection based on immunological methods is one of the important research directions in this field, which may include active immunotherapy (the corresponding drug forms including vaccines, etc.) and passive immunotherapy (the corresponding drug forms including antibodies, etc.). Active immunotherapy refers to stimulating the body of patients with chronic HBV infection to actively produce cellular immune responses (CTL effects, etc.) or/and humoral immune responses (antibodies, etc.) against HBV through the administration of therapeutic vaccines (including protein vaccines, polypeptide vaccines, nucleic acid vaccines, etc.) to achieve the purpose of inhibiting or eliminating HBV. Passive immunotherapy (taking antibodies as an example) refers to passively administering antibodies with therapeutic properties to patients with HBV infection, and using the antibody-mediated virus neutralization to block HBV infection of new liver cells, or using the antibody-mediated immune clearance to eliminate viruses and infected liver cells from the body, thus achieving therapeutic effects.

However, it is known in the art that patients with chronic hepatitis B often develop immune exhaustion (tolerance) against HBV due to high levels of HBsAg in the body, resulting in prolonged infection. It is key to functionally cure CHB to eliminate serum HBsAg, and restore or activate the HBV-specific immunity.

### Contents of the present invention

Although there are multiple B cell response (antibody response) epitopes on various proteins of the HBV virus, not all antibodies against any epitope can be used to treat HBV infection (especially chronic HBV infection). Therefore, the key to develop immunotherapeutic drugs/methods that can effectively treat HBV infection is to identify targets (epitopes) that can induce antibody responses capable of effectively eliminating the virus and virus-infected cells in the body, and to obtain antibodies against the targets (epitopes). The present invention identifies such a target (epitope) and provides an antibody, especially a nanobody, against such epitope peptide/epitope. The nanobody has the advantages of both monoclonal antibody and small molecule drug. It can penetrate the blood-brain barrier, can be easily engineered, developed and stably produced, and has reduced difficulty and cost in the production. The immunotherapy mediated by the epitope peptide or nanobody of the present invention is expected to become a new strategy for CHB patients to combat persistent infection with HBV virus.

### Epitope peptide

In one aspect, the present invention provides an isolated epitope peptide or variant thereof, in which the epitope peptide or variant thereof comprises an epitope located within amino acid residues 157 to 174 of HBsAg protein, the epitope comprises at least amino acid residues 163 to 165 of the HBsAg protein; the variant differs from the epitope peptide from which it is derived only by a mutation (e.g., substitution, addition or deletion) of one or several (e.g., 1, 2, 3, 4, or 5) amino acid residues, and retains the biological function of the epitope peptide from which it is derived.

The biological function of the epitope peptide of the present invention includes, but is not limited to, one or more selected from the following:
1) specifically binding to a nanobody (e.g., 125s) containing CDR1-3 as set forth in SEQ ID NOs: 1-3;
2) reducing a serum level of HBV DNA and/or HBsAg in a subject (optionally, after fusing the epitope peptide with a carrier protein);
3) inducing an antibody response capable of effectively eliminating HBV and HBV-infected cells in a subject, and/or inducing an immune response (e.g., a humoral immune response) against HBV (optionally, after fusing the epitope peptide with a carrier protein); and
4) preventing and/or treating an HBV infection or a disease associated with HBV infection (e.g., hepatitis B) in a subject.

In certain embodiments, the subject in any one of 2) to 4) is an HBV-uninfected subject, or may be an HBV-infected subject, such as a subject with chronic HBV infection. In certain embodiments, the epitope peptide of the present invention has the biological function described in any one of 1) to 4) after being fused to a carrier protein.

In certain embodiments, the epitope located within amino acid residues 157 to 174 of the HBsAg protein comprises at least amino acid residues 163 to 165, and amino acid residues 158, 160 and 171 of the HBsAg protein. In certain embodiments, it further comprises amino acid residue 157.

In certain embodiments, the epitope located within amino acid residues 157 to 174 of the HBsAg protein comprises at least amino acid residues 163 to 166 (e.g., as set forth in SEQ ID NO: 37) of the HBsAg protein.

In certain embodiments, the epitope located within amino acid residues 157 to 174 of the HBsAg protein comprises at least amino acid residues 163 to 166 (e.g., as set forth in SEQ ID NO: 37) and amino acid residues 158, 160 and 171 of the HBsAg protein. In certain embodiments, it further comprises amino acid residue 157.

In certain embodiments, the epitope located within amino acid residues 157 to 174 of the HBsAg protein is a conformational epitope, which consists of at least 3, at least 4, at least 5, at least 6, at least 7, at least 8, at least 9, or at least 10 non-consecutive amino acid residues within amino acid residues 157 to 174 of the HBsAg protein, and comprises at least amino acid residues 163 to 165 of the HBsAg protein.

In certain embodiments, the conformational epitope comprises at least amino acid residues 163 to 165 and amino acid residues 158, 160 and 171 of the HBsAg protein. In certain embodiments, it further comprises amino acid residue 157.

In certain embodiments, the conformational epitope comprises at least amino acid residues 163 to 166 (e.g., as set forth in SEQ ID NO: 37) of the HBsAg protein.

In certain embodiments, the conformational epitope comprises at least amino acid residues 163 to 166 (e.g., as set forth in SEQ ID NO: 37) and amino acid residues 158, 160, and 171 of the HBsAg protein. In certain embodiments, it further comprises amino acid residue 157.

In certain embodiments, the epitope peptide consists of at least 5 (e.g., at least 6, at least 7, at least 8, at least 9, at least 10, at least 11, at least 12, at least 13, at least 14, at least 15, at least 16, at least 17, at least 18, at least 19, at least 20) and/or at most 80 (e.g., at most 75, at most 70, at most 65, at most 60, at most 55, at most 50, at most 45, at most 40, at most 35, at most 30, at most 25, at most 20) consecutive amino acid residues, and comprises the epitope.

In certain embodiments, the epitope peptide consists of 5 to 80 (e.g., 5 to 75, 5 to 70, 5 to 65, 5 to 60, 5 to 55, 5 to 50, 5 to 45, 5 to 40, 5 to 35, 5 to 30, 5 to 25, 5 to 20; such as 5, 6, 7, 8, 9, 10 11, 12, 13, 14, 15, 16, 17, 18, 19, 20, 21, 22, 23, 24, 25, 30, 35, 40, 45, 50, 55, 60, 65, 70, 74, 75 or 80) consecutive amino acid residues, and comprising the epitope.

In certain embodiments, the epitope peptide comprises at least amino acid residues 157 to 174 of the HBsAg protein. In certain embodiments, the amino acid residues 157 to 174 of the HBsAg protein are as set forth in any one of SEQ ID NOs: 19, 38-44.

In certain embodiments, the variant comprises no mutation in positions corresponding to amino acid positions 163 to 165 of the HBsAg protein.

In certain embodiments, the variant comprises no mutation in positions corresponding to amino acid positions 163 to 166 of the HBsAg protein.

In certain embodiments, the variant comprises no mutation in positions corresponding to amino acid positions 162 to 167 of the HBsAg protein.

In certain embodiments, the variant further comprises no mutation in position corresponding to amino acid position 157 of the HBsAg protein.

In certain embodiments, the variant further comprises no mutation in position corresponding to amino acid position 171 of the HBsAg protein.

In certain embodiments, the variant further comprises no mutation in one or more (e.g., 1, 2, or 3) positions corresponding to amino acid positions 158, 160 of the HBsAg protein.

In certain embodiments, the variant comprises a mutation in one or more (e.g., 1, 2, 3, 4, or 5) positions corresponding to amino acid positions 161,162,167,168,169,170,172,173,174 of the HBsAg protein.

In certain embodiments, the mutation is an amino acid substitution, such as a conservative substitution.

In certain embodiments, the amino acid substitution comprises substitution of the amino acid at the position with alanine.

In certain embodiments, the amino acid position given above refers to the position in the sequence as set forth in SEQ ID NO: 36. It is well known to those skilled in the art that the amino acid position can be determined by optimal alignment of the sequences (i.e., when the sequences are aligned to obtain the highest percent identity) to obtain the position in the variant corresponding to the amino acid position described above.

In certain embodiments, the epitope peptide or variant thereof at least comprises the amino acid sequence as set forth in: AX₁X₂X₃X₄X₅WEWAX₆X₇X₈X₉SX₁₀X₁₁X₁₂ (SEQ ID NO: 51); wherein,
X₁ (corresponding to amino acid position 158 of HBsAg protein) is F or L;
X₂ (corresponding to amino acid position 159 of HBsAg protein) is A or G;
X₃ (corresponding to amino acid position 160 of HBsAg protein) is K or R;
X₄ (corresponding to amino acid position 161 of HBsAg protein) is any natural amino acid (e.g., Y, F or A);
X₅ (corresponding to amino acid position 162 of HBsAg protein) is any natural amino acid (e.g., L or A);
X₆ (corresponding to amino acid position 167 of HBsAg protein) is any natural amino acid (e.g., S or A);
X₇ (corresponding to amino acid position 168 of HBsAg protein) is any natural amino acid (e.g., V or A);
X₈ (corresponding to amino acid position 169 of HBsAg protein) is any natural amino acid (e.g., R, H or A);
X₉ (corresponding to amino acid position 170 of HBsAg protein) is any natural amino acid (e.g., F or A);
X₁₀ (corresponding to amino acid position 172 of HBsAg protein) is any natural amino acid (e.g., W or A);
X₁₁ (corresponding to amino acid position 173 of HBsAg protein) is any natural amino acid (e.g., L or A);
X₁₂ (corresponding to amino acid position 174 of the HBsAg protein) is any natural amino acid (e.g., S or A).

In certain embodiments, X₅ is L, X₆ is S, and X₁ to X₄ and X₇ to X₁₂ are as defined above.

In certain embodiments, X₅ is L, X₆ is S, X₉ is F, X₁₀ is W, X₁₁ is L, X₁₂ is S, and X₁ to X₄ and X₇ to X₈ are as defined above.

In certain embodiments, the epitope peptide or variant thereof at least comprises an amino acid sequence selected from the following: the sequences as set forth in any one of SEQ ID NOs: 19, 38-44.

In certain embodiments, the HBsAg protein comprises the sequence as set forth in SEQ ID NO: 36, or a sequence having a sequence identity of at least 80%, at least 85%, at least 90%, at least 91%, at least 92%, at least 93% compared to at least 94%, at least 95%, at least 96%, at least 97%, at least 98%, at least 99%, or 100% as compared thereto.

In certain embodiments, the epitope peptide or variant thereof comprises or consists of the sequence as set forth in any one of SEQ ID NOs: 16, 19, 22-35, 38-44.

### Recombinant protein

The epitope peptide or variant thereof of the present invention can be fused to a carrier protein to enhance the immunogenicity of the epitope peptide or variant thereof, and/or to maintain the conformational epitope contained in the epitope peptide or variant thereof, so that it can be recognized by the body's immune system and induce the body's immune response.

Therefore, in another aspect, the present invention provides a recombinant protein, which comprises the isolated epitope peptide or variant thereof as described above, and a carrier protein, and the recombinant protein is not a naturally occurring protein or fragment thereof. In the recombinant protein, the epitope peptide or variant thereof can be linked to the N-terminal or C-terminal of the carrier protein, be inserted into the interior of the carrier protein, or replace part of the amino acid sequence of the carrier protein, depending on the specific carrier protein as used. Furthermore, optionally, the epitope peptide or variant thereof is linked to the carrier protein via a linker (e.g., a rigid or flexible linker, such as a peptide linker comprising one or more glycines and/or one or more serines). The recombinant protein of the present invention is not limited by its production method. For example, it can be produced by a genetic engineering method (recombinant technology) or by a chemical synthetic method.

In certain embodiments, the biological function of the recombinant protein includes, but is not limited to, one or more selected from the following: 1) specifically binding to a nanobody (e.g., 125s) containing CDR1-3 as set forth in SEQ ID NOs: 1-3; 2) reducing a serum level of HBV DNA and/or HBsAg in a subject; 3) inducing an antibody response capable of effectively eliminating HBV and HBV-infected cells in a subject, and/or inducing an immune response (e.g., a humoral immune response) against HBV; and 4) preventing and/or treating an HBV infection or a disease associated with HBV infection (e.g., hepatitis B) in a subject.

In certain embodiments, the carrier protein is selected from an immunoglobulin Fc domain, such as an IgG Fc domain (e.g., an Fc domain of IgG1, IgG2, IgG3, or IgG4).

In certain embodiments, the epitope peptide or variant thereof is optionally linked to the N-terminal or C-terminal (e.g., N-terminal) of an immunoglobulin Fc domain by a linker (e.g., a rigid or flexible linker, such as a peptide linker comprising one or more glycines and/or one or more serines). In certain embodiments, the immunoglobulin Fc domain comprises the sequence as set forth in SEQ ID NO: 14.

In certain embodiments, the carrier protein is selected from a protein capable of self-assembling to form a nanoparticle, whereby the recombinant protein is capable of forming an antigenic nanoparticle. The antigen nanoparticle is an assembly of polypeptide that present multiple copies of an antigen (e.g., the epitope peptide or variant thereof of the present invention), which results in multiple binding sites (affinities) and may provide improved antigen stability and immunogenicity. The preparation of self-assembling protein nanoparticle and use thereof in vaccines are well known to those skilled in the art. As non-limiting examples, self-assembling nanoparticle can be based on Ferritin, Encapsulin or Lumazine synthase (LS).

In certain embodiments, the protein capable of self-assembling to form nanoparticle is ferritin, such as murine ferritin.

In certain embodiments, the epitope peptide or variant thereof is linked to the N-terminal or C-terminal (e.g., N-terminal) of the protein capable of self-assembling to form a nanoparticle via a peptide bond or an isopeptide bond.

In certain embodiments, the epitope peptide or variant thereof is linked to the N-terminal or C-terminal (e.g., N-terminal) of the protein capable of self-assembling to form a nanoparticle via an isopeptide bond.

In certain embodiments, the isopeptide bond is formed by a protein-protein binding pair.

In certain embodiments, the protein-protein binding pair consists of a first member (e.g., a first peptide tag) and a second member (e.g., a second peptide tag), wherein the first member and the second member are linked via an isopeptide bond, and, the first member is linked to the N-terminal or C-terminal of the epitope peptide or variant thereof optionally via a first linker (e.g., a rigid or flexible linker, such as a peptide linker comprising one or more glycines and/or one or more serines) to form a first protein, and the second member is linked to the N-terminal or C-terminal (e.g., N-terminal) of the protein capable of self-assembling to form nanoparticle optionally via a second linker (e.g., a rigid or flexible linker, such as one or more glycines and/or one or more Serine peptide linker) to form a second protein.

In certain embodiments, the first protein and/or second protein may further comprise a protein tag (e.g., at N-terminal or C-terminal thereof).

In certain embodiments, the protein-protein binding pair is selected from: (i) SpyTag:SpyCatcher pair, (ii) SpyTag:KTag pair, (iii) Isopeptag:pilin-C pair, (iv) SnoopTag or SnoopTagJr:SnoopCatcher pair, (v) SpyTag002:SpyCatcher002 pair, (vi) RrgATag, RrgATag2 or DogTag:RrgACatcher pair, (vii) IsopepTag N:Pilin N pair, (viii) PsCsTag:PsCsCatcher pair.

In certain embodiments, the protein-protein binding pair is a SpyTag: SpyCatcher pair; for example, the SpyTag comprises the sequence as set forth in SEQ ID NO: 46; for example, the SpyCatcher comprises the sequence as set forth in SEQ ID NO: 47 or 48.

In certain embodiments, the recombinant protein of the present invention is formed by linking a first protein and a second protein via an isopeptide bond, wherein:
the first protein comprises the epitope peptide or variant thereof and a first member of SpyTag: SpyCatcher pair, and the first member is linked to the epitope peptide or variant thereof (e.g., linked to the N-terminal or C-terminal of the epitope peptide or variant thereof) optionally via a first linker;
the second protein comprises the protein capable of self-assembling to form nanoparticle and a second member of SpyTag: SpyCatcher pair, and the second member is linked to the protein capable of self-assembling to form nanoparticle (e.g., linked to the N-terminal of the protein capable of self-assembling to form nanoparticle) optionally via a second linker.

In certain embodiments, the first protein comprises the epitope peptide or variant thereof and SpyCatcher or an active fragment thereof (e.g., an N-terminal truncation), and the second protein comprises the protein capable of self-assembling to form nanoparticle and SpyTag.

In certain embodiments, the first protein comprises the sequence as set forth in SEQ ID NO:49, and the second protein comprises the sequence as set forth in SEQ ID NO:50.

### Preparation of epitope peptide and recombinant protein

In another aspect, the present invention further provides an isolated nucleic acid molecule, which comprises a nucleotide sequence encoding the epitope peptide or variant thereof of the present invention or the recombinant protein of the present invention.

In certain embodiments, the isolated nucleic acid molecule encodes the epitope peptide or variant thereof of the present invention.

In certain embodiments, the isolated nucleic acid molecule encodes the recombinant protein of the present invention.

In certain embodiments, the recombinant protein encoded by the isolated nucleic acid molecule is formed by linking a first protein and a second protein via an isopeptide bond, wherein, the first protein comprises the epitope peptide or variant thereof and a first member of the protein-protein binding pair; the second protein comprises the protein capable of self-assembling to form nanoparticle and a second member of the protein-protein binding pair. In such embodiments, the isolated nucleic acid molecule comprises a first nucleotide sequence encoding the first protein and a second nucleotide sequence encoding the second protein, and the first nucleotide sequence and the second nucleotide sequence are present on the same or different isolated nucleic acid molecules.

In another aspect, the present invention also provides a vector, which comprises the isolated nucleic acid molecule as described above. The vector of the present invention may be a cloning vector or an expression vector. In certain embodiments, the vector of the present invention is, for example, a plasmid, a cosmid, a phage, a cosmid, and the like. In certain embodiments, the vector is capable of expressing the epitope peptide or variant thereof of the present invention or the recombinant protein of the present invention in a subject (e.g., a mammal, such as a human).

In certain embodiments, the vector encodes the epitope peptide or variant thereof of the present invention.

In certain embodiments, the vector encodes the recombinant protein of the present invention.

In certain embodiments, the recombinant protein encoded by the vector is formed by linking a first protein and a second protein through an isopeptide bond, wherein: the first protein comprises the epitope peptide or variant thereof and a first member of the protein-protein binding pair; the second protein comprises the protein capable of self-assembling to form nanoparticle and a second member of the protein-protein binding pair. In such embodiments, the vector comprises a first nucleotide sequence encoding the first protein and a second nucleotide sequence encoding the second protein, the first nucleotide sequence and the second nucleotide sequence are present on the same or different vectors.

In another aspect, the present invention further provides a host cell comprising the isolated nucleic acid molecule or vector of the present invention. Such host cell includes, but is not limited to, prokaryotic cell such as bacterial cell (e.g., *E. coli* cell), and eukaryotic cell such as fungal cell (e.g., yeast cell), insect cell, plant cell, and animal cell (e.g., mammalian cell, such as mouse cell, human cell, etc.). The cell of the present invention may also be a cell line, such as HEK293 cell. In certain embodiments, the host cell is a microorganism, such as a bacterial cell or a fungal cell.

In another aspect, the present invention further provides a method for preparing the epitope peptide or variant thereof or the recombinant protein of the present invention, which comprises culturing the host cell of the present invention under suitable conditions, and recovering the epitope peptide or variant thereof or the recombinant protein of the present invention from a cell culture.

### Multimer

In another aspect, the present invention provides a multimer comprising a plurality of monomers, wherein each monomer is independently selected from the recombinant protein of the present invention.

In certain embodiments, the multimer is a dimer, trimer, or tetramer.

In certain embodiments, the monomers are identical to each another.

In certain embodiments, the monomers are identical to each other, and are selected from the recombinant protein comprising the epitope peptide or variant thereof of the present invention and an immunoglobulin Fc domain as a carrier protein.

In certain embodiments, the biological function of the multimer includes, but is not limited to, one or more selected from the following: 1) specifically binding to a nanobody (e.g., 125s) containing CDR1-3 as set forth in SEQ ID NOs: 1-3; 2) reducing a serum level of HBV DNA and/or HBsAg in a subject; 3) inducing an antibody response capable of effectively eliminating HBV and HBV-infected cells in a subject, and/or inducing an immune response (e.g., a humoral immune response) against HBV; and 4) preventing and/or treating an HBV infection or a disease associated with HBV infection (e.g., hepatitis B) in a subject.

### Particle

In another aspect, the present invention provides a particle, displaying on its surface the isolated epitope peptide or variant thereof of the present invention.

In certain embodiments, the particle is a nanoparticle, a virus-like particle (VLP) or a core-like particle (CLP).

In certain embodiments, the particle comprises or consists of the recombinant protein or multimer of the present invention.

In certain embodiments, the particle is a nanoparticle formed by a recombinant protein comprising the epitope peptide or variant thereof of the present invention and a protein capable of self-assembling to form nanoparticle as a carrier protein.

In certain embodiments, the biological function of the particle includes, but is not limited to, one or more selected from the following: 1) specifically binding to a nanobody (e.g., 125s) containing CDR1-3 as set forth in SEQ ID NOs: 1-3; 2) reducing a serum level of HBV DNA and/or HBsAg in a subject; 3) inducing an antibody response capable of effectively eliminating HBV and HBV-infected cells in a subject, and/or inducing an immune response (e.g., a humoral immune response) against HBV; and 4) preventing and/or treating an HBV infection or a disease associated with HBV infection (e.g., hepatitis B) in a subject.

### Use of epitope peptide

In another aspect, the present invention provides a pharmaceutical composition, which comprises the epitope peptide or variant thereof, the recombinant protein, the isolated nucleic acid molecule, the vector, the host cell, the multimer or the particle as described above.

In certain embodiments, the pharmaceutical composition is a vaccine.

In certain embodiments, the pharmaceutical composition further comprises a pharmaceutically acceptable carrier and/or excipient (e.g., adjuvant). In certain embodiments, the pharmaceutically acceptable carrier and/or excipient comprises an adjuvant. Adjuvant for co-administration or comprised in the pharmaceutical compositions of the present invention should preferably be an adjuvant that is potentially safe, well tolerated and effective in human body. Such adjuvants are well known to those skilled in the art.

In certain embodiments, the pharmaceutical composition is a protein vaccine, which comprises the epitope peptide or variant thereof, the recombinant protein, the multimer or the particle as described above. In certain embodiments, the protein vaccine comprises one or more of the epitope peptides or variants thereof of the present invention, and these epitope peptides or variants thereof may be separate or in tandem, modified or unmodified, coupled to other proteins, or not coupled to other proteins.

In certain embodiments, the pharmaceutical composition is a nucleic acid vaccine, which comprises the isolated nucleic acid molecule or the vector as described above. In certain embodiments, the nucleic acid vaccine comprises DNA or RNA. In such embodiments, the DNA or RNA may be naked or may be encapsulated in a shell with delivery and/or protection functions. In some embodiments, the shell can be a shell of adenovirus, adeno-associated virus, lentivirus, retrovirus, etc., or other material that is synthesized by chemical method and can perform similar functions.

The epitope peptide or variant thereof, recombinant protein, multimer, particle, or pharmaceutical composition as described above can be formulated into any dosage form known in the medical field, for example, tablet, pill, suspension, emulsion, solution, gel, capsule, powder, granule, elixir, lozenge, suppository, injection (including injection solution, sterile powder for injection and concentrated solution for injection), inhalant, spray, etc. The preferred dosage form depends on the intended mode of administration and therapeutic use. The epitope peptide or variant thereof, recombinant protein, multimer, particle, or pharmaceutical composition as described above should be sterile and stable under the conditions of production and storage. One preferred dosage form is an injection. Such injection may be a sterile injectable solution. Additionally, sterile injectable solution may be prepared as sterile lyophilized powder (e.g., by vacuum drying or freeze drying) for ease of storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution and any combination thereof.

The epitope peptide or variant thereof, recombinant protein, multimer, particle, or pharmaceutical composition as described above can be administered by any suitable method known in the art, including but not limited to, oral, buccal, sublingual, ocular, topical, parenteral, rectal, intrathecal, intracytoplasmic reticulum, inguinal, intravesical, topical (e.g., powder, ointment, or drops), or nasal route. However, for many therapeutic uses, the preferred route/mode of administration is parenteral administration (e.g., intravenous or bolus injection, subcutaneous injection, intraperitoneal injection, intramuscular injection). The skilled artisan will understand that the route and/or mode of administration will vary depending on the intended purpose.

The epitope peptide or variant thereof, recombinant protein, multimer, particle, or pharmaceutical composition as described above should be administered in an effective dose. The appropriate amount can be determined according to the specific disease or condition to be treated or prevented, the severity thereof, the age of subject, and other personal attributes of the particular subject (e.g., the general state of the subject's health and the state of the subject's immune system). The determination of effective dose is also guided by studies in animal models, followed by human clinical trials, and by administration regimens that significantly reduce the occurrence or severity of symptoms or conditions of the target disease in subjects.

In another aspect, the present invention provides a use of the epitope peptide or variant thereof, recombinant protein, isolated nucleic acid molecule, vector, host cell, multimer, particle, or pharmaceutical composition as described above in the manufacture of a preparation, and the preparation is used for reducing serum levels of HBV DNA and/or HBsAg in a subject (e.g., a human), inducing an immune response (e.g., a humoral immune response) against HBV in a subject (e.g., a human), and/ or preventing and/or treating HBV infection or a disease associated with HBV infection (e.g., hepatitis B) in a subject (e.g., a human).

In certain embodiments, the preparation is a vaccine.

In certain embodiments, the preparation is a protein vaccine. In certain embodiments, the present invention provides a use of the epitope peptide or variant, recombinant protein, multimer or particle as described above in the manufacture of a protein vaccine, and the protein vaccine is used for reducing serum levels of HBV DNA and/or HBsAg in a subject (e.g., a human), inducing an immune response (e.g., a humoral immune response) against HBV in a subject (e.g., a human), and/ or preventing and/or treating HBV infection or a disease associated with HBV infection (e.g., hepatitis B) in a subject (e.g., a human).

In certain embodiments, the preparation is a nucleic acid vaccine. In certain embodiments, the present invention provides a use of the nucleic acid molecule or vector as described above in the manufacture of a nucleic acid vaccine, and the nucleic acid vaccine is used for reducing serum levels of HBV DNA and/or HBsAg in a subject (e.g., a human), inducing an immune response (e.g., a humoral immune response) against HBV in a subject (e.g., a human), and/ or preventing and/or treating HBV infection or a disease associated with HBV infection (e.g., hepatitis B) in a subject (e.g., a human).

In certain embodiments, the subject is an HBV-uninfected subject. In certain embodiments, the subject is a subject infected with HBV, such as a subject with chronic HBV infection or a chronic hepatitis B patient.

In another aspect, the present invention provides a method for reducing serum levels of HBV DNA and/or HBsAg in a subject (e.g., a human), inducing an immune response against HBV in a subject (e.g., a human), and/ or preventing and/or treating HBV infection or a disease associated with HBV infection (e.g., hepatitis B) in a subject (e.g., a human), which comprises: administering to a subject in need thereof an effective amount of the epitope peptide or variant thereof, recombinant protein, isolated nucleic acid molecule, vector, host cell, multimer, particle, or pharmaceutical composition as described above. In certain embodiments, the subject is an HBV-uninfected subject. In certain embodiments, the subject is a subject infected with HBV, such as a subject with chronic HBV infection or a chronic hepatitis B patient.

For prophylactic applications, the epitope peptide or variant thereof, recombinant protein, isolated nucleic acid molecule, vector, host cell, multimer, particle, or pharmaceutical composition as described above may be used before any symptoms appear, for example before infection. Prophylactic administration is used to prevent or ameliorate any subsequent infection, to attenuate the expected severity, duration, or degree of infection and/or associated disease symptoms after exposure or suspected exposure to virus or after actual onset of infection. In some embodiments, the subject to be treated may be a subject at risk of suffering HBV infection, for example, due to exposure or potential exposure to HBV.

For therapeutic applications, the epitope peptide or variant thereof, recombinant protein, isolated nucleic acid molecule, vector, host cell, multimer, particle, or pharmaceutical composition as described above may be administered at or after the onset of symptoms of disease or infection, for example, provided after the onset of symptoms of HBV infection or after diagnosis of HBV infection. In some embodiments, the subject to be treated can also be a subject infected with HBV, such as a subject with chronic HBV infection or a chronic hepatitis B patient.

In certain embodiments, the subject is administered with a protein vaccine comprising the epitope peptide or variant thereof, recombinant protein, multimer or particle as described above.

In certain embodiments, the subject is administered with a nucleic acid vaccine comprising the isolated nucleic acid molecule or vector as described above.

### Nanobody and derivative thereof

In another aspect, the present invention provides a nanobody or antigen-binding fragment thereof capable of specifically binding to HBsAg. The nanobody generally consists of 4 framework regions (FRs) and 3 complementarity determining regions (CDRs), called FR1, CDR1, FR2, CDR2, FR3, CDR3 and FR4, and the antigen-binding fragment comprises at least a part of the nanobody, which part is sufficient to confer the fragment with the ability to specifically bind HBsAg. In some embodiments, the nanobody of the present invention can be truncated at the N- or C-terminal so that it contains only part of FR1 and/or FR4, or lacks one or two of the framework regions, as long as it substantially maintains the ability to bind antigen and the specificity.

In certain embodiments, the nanobody or antigen-binding fragment thereof is capable of specifically binding to the epitope peptide or variant thereof or epitope contained therein, recombinant protein, multimer or particle as described above.

In certain embodiments, the nanobody or antigen-binding fragment thereof comprises: CDR1 having the sequence as set forth in SEQ ID NO: 1 or a sequence having a substitution, deletion or addition of one or several amino acids (e.g., a substitution, deletion or addition of 1, 2 or 3 amino acids) as compared thereto; CDR2 having the sequence as set forth in SEQ ID NO: 2 or a sequence having a substitution, deletion or addition of one or several amino acids (e.g., a substitution, deletion or addition of 1, 2 or 3 amino acids) as compared thereto; and, CDR3 having the sequence as set forth in SEQ ID NO: 3 or a sequence having a substitution, deletion or addition of one or several amino acids (e.g., a substitution, deletion or addition of 1, 2 or 3 amino acids) as compared thereto.

In certain embodiments, the nanobody or antigen-binding fragment thereof further comprises: FR1 having the sequence as set forth in SEQ ID NO: 4 or a sequence having a substitution, deletion or addition of one or several amino acids (e.g., a substitution, deletion or addition of 1, 2 or 3 amino acids) as compared thereto; FR2 having the sequence as set forth in SEQ ID NO: 5 or a sequence having a substitution, deletion or addition of one or several amino acids (e.g., a substitution, deletion or addition of 1, 2 or 3 amino acids) as compared thereto; FR3 having the sequence as set forth in SEQ ID NO: 6 or a sequence having a substitution, deletion or addition of one or several amino acids (e.g., a substitution, deletion or addition of 1, 2 or 3 amino acids) as compared thereto; and, FR4 having the sequence as set forth in SEQ ID NO: 7 or a sequence having a substitution, deletion or addition of one or several amino acids (e.g., a substitution, deletion or addition of 1, 2 or 3 amino acids) as compared thereto.

In certain embodiments, the nanobody or antigen-binding fragment thereof comprises the sequence as set forth in SEQ ID NO: 8, or a sequence having a sequence identity of at least 80%, at least 85%, at least 90%, at least 91%, or at least 92%, at least 93%, at least 94%, at least 95%, at least 96%, at least 97%, at least 98%, at least 99%, or 100% as compared thereto, or a sequence having a substitution, deletion or addition of one or several amino acids (e.g., a substitution, deletion or addition of 1, 2, 3, 4 or 5 amino acids) as compared thereto. In certain embodiments, the substitution is a conservative substitution.

In certain embodiments, the nanobody or antigen-binding fragment thereof is humanized. In certain embodiments, the nanobody or antigen-binding fragment thereof is a humanized VHH, i.e., a VHH in which one or more framework regions have been substantially replaced with human framework regions.

In certain embodiments, the nanobody or antigen-binding fragment thereof further comprises a heavy chain framework region of a human immunoglobulin (e.g., a heavy chain framework region contained in the amino acid sequence encoded by a human heavy chain germline antibody gene), and the heavy chain framework region optionally comprises one or more (e.g., 1, 2, 3, 4, 5, 6, 7, 8, 9 or 10) back mutations from human residues to camel residues.

In certain embodiments, the nanobody or antigen-binding fragment thereof comprises: FR1 to FR3 derived from IGHV3-23*04 germline gene sequence and FR4 derived from IGHJ5*02 germline gene sequence, the FR1 to FR4 optionally comprises one or more (e.g., 1, 2, 3, 4, 5, 6, 7, 8, 9, or 10) back mutations from human residues to camel residues.

In certain embodiments, the nanobody or antigen-binding fragment thereof comprises: FR1 having the sequence as set forth in SEQ ID NO: 9 or a sequence having a substitution, deletion or addition of one or several amino acids (e.g., a substitution, deletion or addition of 1, 2 or 3 amino acids) as compared thereto; FR2 having the sequence as set forth in SEQ ID NO: 10 or a sequence having a substitution, deletion or addition of one or several amino acids (e.g., a substitution, deletion or addition of 1, 2 or 3 amino acids) as compared thereto; FR3 having the sequence as set forth in SEQ ID NO: 11 or a sequence having a substitution, deletion or addition of one or several amino acids (e.g., a substitution, deletion or addition of 1, 2 or 3 amino acids) as compared thereto; and, FR4 having the sequence as set forth in SEQ ID NO: 12 or a sequence having a substitution, deletion or addition of one or several amino acids (e.g., a substitution, deletion or addition of 1, 2 or 3 amino acids) as compared thereto.

In certain embodiments, the nanobody or antigen-binding fragment thereof comprises the sequence as set forth in SEQ ID NO: 13, or a sequence having a sequence identity of at least 80%, at least 85%, at least 90%, at least 91%, or at least 92%, at least 93%, at least 94%, at least 95%, at least 96%, at least 97%, at least 98%, at least 99%, or 100% as compared thereto, or a sequence having a substitution, deletion or addition of one or several amino acids (e.g., a substitution, deletion or addition of 1, 2, 3, 4 or 5 amino acids) as compared thereto. In certain embodiments, the substitution is a conservative substitution.

In certain embodiments, the nanobody or antigen-binding fragment thereof or polypeptide construct of the present invention is capable of specifically binding to HBsAg, neutralizing the virulence of HBV, reducing the serum levels of HBV DNA and/or HBsAg in a subject, and/or activating the humoral immune response against HBV in a subject (e.g., a subject with chronic HBV infection, or a chronic hepatitis B patient). In certain embodiments, the HBV comprises genotypes A, B, C, D, E, F, G, H, I, J. The genotype of HBV can be found in the NCBI taxonomy database (Taxonomy), with the accession number txid10407.

The nanobody or antigen-binding fragment thereof of the present invention may be derivatized, for example linked to another molecule (e.g., another polypeptide or protein). Generally, derivatization (e.g., labeling) of the antibody or antigen-binding fragment thereof will not adversely affect its binding to HBsAg. Therefore, the nanobody or antigen-binding fragment thereof of the present invention is intended to include such derivatized form. For example, the nanobody or antigen-binding fragment thereof of the present invention can be functionally linked (by chemical coupling, genetic fusion, non-covalent linkage, or other means) to one or more other molecular groups, such as another antibody (e.g., to form a bispecific antibody), an additional functional peptide (e.g., Fc domain), a detection reagent, a pharmaceutical reagent, and/or a protein or peptide capable of mediating the binding of the antibody or antigen-binding fragment to another molecule (e.g., an antibiotin protein or a polyhistidine tag).

Therefore, in another aspect, the present invention further provides a polypeptide construct comprising the nanobody or antigen-binding fragment thereof of the present invention and an additional polypeptide, and the polypeptide construct is capable of specifically binding to HBsAg.

In certain embodiments, the polypeptide construct comprises the nanobody or antigen-binding fragment thereof of the present invention, and an immunoglobulin Fc domain.

In this context, the Fc domain, also called Fc region, refers to a part of heavy chain constant region that contains CH2 and CH3. In some embodiments, the Fc domain comprises a hinge, CH2, and CH3. When the Fc domain comprises a hinge, the hinge mediates the dimerization between the two Fc-containing polypeptides. The Fc domain can be of any isotype of antibody heavy chain constant region. In some embodiments, the Fc domain is IgGl, IgG2, IgG3 or IgG4.

In certain embodiments, the Fc domain contained in the polypeptide construct of the present invention is a native Fc region, which comprises an amino acid sequence consistent with the amino acid sequence of an Fc region found in nature. For example, the Fc domain may be a human IgG1 Fc region with native sequence, human IgG2 Fc region with native sequence, human IgG3 Fc region with native sequence, or human IgG4 Fc region with native sequence. Native Fc regions can have effector functions. Exemplary "effector functions" include binding to Fc receptors; Clq binding and complement-dependent cytotoxicity (CDC); antibody-dependent cell-mediated cytotoxicity (ADCC); phagocytosis; downregulation for cell surface receptor (e.g., B cell receptor); and B cell activation, etc. Functional changes can be produced by replacing at least one amino acid residue in the native Fc region with a different residue or by chemical modification, for example, changing the affinity of antibody for an effector ligand (e.g., FcR or complement C1q), thus altering effector function (e.g., reduce or enhance).

Therefore, in certain embodiments, the Fc domain contained in the polypeptide construct of the present invention may also be a variant Fc region, which may comprises a mutation of one or more (e.g., 1 to 10, such as 1 to 5) amino acids as compared to the native Fc region, or be chemically modified to change one or more of the following properties of the antibody of the present invention: Fc receptor binding, antibody glycosylation, number of cysteine residues, effector cell function, or complement function, etc..

In certain embodiments, the immunoglobulin Fc domain is linked to the N-terminal and/or C-terminal of the nanobody or antigen-binding fragment thereof optionally via a peptide linker. In certain embodiments, the immunoglobulin Fc domain is linked to the C-terminal of the nanobody or antigen-binding fragment thereof optionally via a peptide linker.

In certain embodiments, the immunoglobulin Fc domain is an IgG Fc domain (e.g., an IgG1, IgG2, IgG3, or IgG4 Fc domain).

In certain embodiments, the immunoglobulin Fc domain is a human immunoglobulin Fc domain, such as an Fc domain of human IgG (e.g., an Fc domain of human IgG1, IgG2, IgG3, or IgG4). In certain embodiments, the immunoglobulin Fc domain comprises the sequence as set forth in SEQ ID NO: 14, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereof, or a sequence having a substitution, deletion or addition of one or several amino acids (e.g., a substitution, deletion or addition of 1, 2, 3, 4 or 5 amino acids) as compared thereto. In certain embodiments, the substitution is a conservative substitution.

In certain embodiments, the immunoglobulin Fc domain is a murine immunoglobulin Fc domain, such as an Fc domain of murine IgG (e.g., an Fc domain of murine IgG1, IgG2, IgG3, or IgG4). In certain embodiments, the immunoglobulin Fc domain comprises the sequence as set forth in SEQ ID NO: 15, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereof, or a sequence having a substitution, deletion or addition of one or several amino acids (e.g., a substitution, deletion or addition of 1, 2, 3, 4 or 5 amino acids) as compared thereto. In certain embodiments, the substitution is a conservative substitution.

In another aspect, the present invention also provides a conjugate, which comprises the nanobody or antigen-binding fragment thereof or polypeptide construct of the present invention and a conjugating moiety.

In certain embodiments, the conjugating moiety is a detectable label, such as an enzyme, a radionuclide, a fluorescent dye, a luminescent substance (e.g., a chemiluminescent substance), or a biotin. The detectable label of the present invention can be any substance detectable by fluorescent, spectroscopy, photochemical, biochemical, immunological, electrical, optical or chemical means. Such labels are well known in the art and examples thereof include, but are not limited to, enzyme (e.g., horseradish peroxidase, alkaline phosphatase, beta-galactosidase, urease, glucose oxidase, etc.), radionuclide (e.g., ³H, ¹²⁵I, ³⁵S, ¹⁴C, or ³²P), fluorescent dye (e.g., fluorescein isothiocyanate (FITC), fluorescein, tetramethylrhodamine isothiocyanate (TRITC), phycoerythrin (PE), Texas Red, rhodamine, quantum dots or cyanine dye derivative (e.g., Cy7, Alexa 750)), luminescent substance (e.g., chemiluminescent substance such as acridinium ester), magnetic beads (e.g., Dynabeads^{®}), calorimetric label such as colloidal gold or colored glass or plastic (e.g., polystyrene, polypropylene, latex, etc.) beads, and biotin for binding to avidin (e.g., streptavidin) modified by the above labels. In certain embodiments, such labels are suitable for immunological detection (e.g., enzyme-linked immunoassay, radioimmunoassay, fluorescent immunoassay, chemiluminescent immunoassay, etc.). In certain embodiments, the detectable label as described above can be linked to the nanobody or antigen-binding fragment thereof or polypeptide construct of the present invention via a linker of different lengths to reduce potential steric hindrance.

In certain embodiments, the conjugating moiety is a therapeutic agent.

### Preparation of antibody and derivative thereof

The antibody or polypeptide construct of the present invention can be prepared by various methods known in the art, such as by genetic engineering recombinant technology. For example, a DNA molecule encoding the antibody or polypeptide construct of the present invention is obtained by chemical synthesis or PCR amplification. The resulting DNA molecule is inserted into an expression vector and then transfected into a host cell. Then, the transfected host cell is cultured under specific conditions and expresses the antibody or polypeptide construct of the present invention.

Accordingly, in another aspect, the present invention provides an isolated nucleic acid molecule, which comprising a nucleotide sequence encoding the nanobody or antigen-binding fragment thereof or polypeptide construct of the present invention. In certain embodiments, the isolated nucleic acid molecule encodes the nanobody or antigen-binding fragment thereof or polypeptide construct of the present invention.

In another aspect, the present invention provides a vector (e.g., a cloning vector or an expression vector), which comprises the isolated nucleic acid molecule as described above. In certain embodiments, the vector of the present invention is, for example, a plasmid, a cosmid, a phage, and the like.

In another aspect, the present invention provides a host cell, which comprises the isolated nucleic acid molecule or vector as described above. Such host cell includes, but is not limited to, prokaryotic cell such as bacterial cell (e.g., *E. coli* cell), and eukaryotic cell such as fungal cell (e.g., yeast cell), insect cell, plant cell, and animal cell (e.g., mammalian cell, such as mouse cell, human cell, etc.). In certain embodiments, the host cell of the present invention is a mammalian cell, such as HEK293. In certain embodiments, the host cell is a microorganism, such as a bacterial cell or a fungal cell.

In another aspect, the present invention further provides a method for preparing the nanobody or antigen-binding fragment thereof of the present invention, which comprises culturing the host cell as described above under conditions that allow protein expression, and recovering the nanobody or antigen-binding fragment thereof or polypeptide construct from a culture of the cultured host cell.

### Therapeutic use of antibody

In another aspect, the present invention provides a pharmaceutical composition, which comprises the nanobody or antigen-binding fragment thereof or polypeptide construct of the present invention, and a pharmaceutically acceptable carrier and/or excipient.

In certain embodiments, the nanobody or antigen-binding fragment thereof or polypeptide construct of the present invention may be used in combination with an additional pharmaceutically active agent. Therefore, the pharmaceutical composition of the present invention may further comprise an additional pharmaceutically active agent, such as a drug for preventing or treating HBV infection or a disease related to HBV infection (e.g., hepatitis B), such as interferon drug, such as interferon or pegylated interferon.

In another aspect, there is provided a use of the nanobody or antigen-binding fragment thereof or polypeptide construct of the present invention or the pharmaceutical composition of the present invention in the manufacture of a medicament for preventing and/or treating HBV infection or a disease associated with HBV infection (e.g., hepatitis B) in a subject (e.g., a human), for neutralizing HBV virulence in vitro or in a subject (e.g., a human), for reducing serum levels of HBV DNA and/or HBsAg HBV in a subject (e.g., a human), and/or for activating a humoral immune response against HBV in a subject (e.g., a subject with chronic HBV infection or a chronic hepatitis B patient).

In another aspect, the present invention provides a method, which is used for preventing or treating HBV infection or a disease associated with HBV infection (e.g., hepatitis B) in a subject (e.g., a human), for neutralizing HBV virulence in vitro or in a subject (e.g., a human), for reducing serum levels of HBV DNA and/or HBsAg HBV in a subject (e.g., a human), and/or for activating a humoral immune response against HBV in a subject (e.g., a subject with chronic HBV infection or a chronic hepatitis B patient), and the method comprises administering to the subject in need thereof an effective amount of the nanobody or antigen-binding fragment thereof or polypeptide construct of the present invention or the pharmaceutical composition of the present invention.

In any of the above aspects, the subject may be a subject not infected with HBV, or the subject may be a subject infected with HBV, such as a subject with chronic HBV infection or a chronic hepatitis B patient.

In any of the above aspects, the HBV involved in the nanobody or antigen-binding fragment thereof or polypeptide construct or the pharmaceutical composition of the present invention comprises but is not limited to genotype A, B, C, D, E, F, G, H, I, J.

The method of administration of the nanobody or antigen-binding fragment thereof or polypeptide construct of the present invention or the pharmaceutical composition of the present invention can be a traditional administration route, including but not limited to, oral, buccal, sublingual, ocular, topical, parenteral, rectal, intrathecal, intracytoplasmic reticulum, inguinal, intravesical, topical (e.g., powder, ointment, or drops), or nasal route. The nanobody or antigen-binding fragment thereof or polypeptide constructs thereof of the present invention can be administered by a variety of methods known in the art. However, for many therapeutic uses, the preferred route/mode of administration is parenteral (e.g., intravenous injection, subcutaneous injection, intraperitoneal injection, intramuscular injection). The skilled artisan will understand that the route and/or mode of administration will vary depending on the intended purpose. In certain embodiments, the nanobody or antigen-binding fragment thereof or polypeptide construct of the present invention is administered by intravenous infusion or injection.

The nanobody or antigen-binding fragment thereof or polypeptide construct of the present invention or the pharmaceutical composition of the present invention can be formulated into various dosage forms, such as liquid, semi-solid and solid dosage forms, such as solution (e.g., injection), dispersion or suspension, tablet, powder, granule, emulsion, pill, syrup, powder, liposome, capsule and suppository. The preferred dosage form depends on the intended mode of administration and therapeutic use.

For example, one of the preferred dosage forms is an injection. Such injection may be a sterile injectable solution. For example, the sterile injectable solution can be prepared by the following method: incorporating in an appropriate solvent a necessary dose of the nanobody or antigen-binding fragment thereof or polypeptide construct of the present invention, and, optionally, other desired ingredients (including, but not limited to, pH adjuster, surfactant, adjuvant, ionic strength enhancer, isotonic agent, preservative, diluent, or any combination thereof), followed by filtration for sterilization. Additionally, the sterile injectable solution may be prepared as a sterile lyophilized powder (e.g., by vacuum drying or freeze drying) for ease of storage and use. Such sterile lyophilized powder can be dispersed in a suitable carrier, such as sterile pyrogen-free water, before use.

Furthermore, the nanobody or antigen-binding fragment thereof or polypeptide construct of the present invention may be present in unit dosage form in a pharmaceutical composition to facilitate administration. The pharmaceutical composition of the present invention should be sterile and stable under the conditions of production and storage.

The nanobodies or antigen-binding fragments thereof or polypeptide constructs thereof or the pharmaceutical compositions provided by the present invention can be used alone or in combination, or used in combination with an additional pharmaceutically active agent (e.g., an additional antiviral agent, such as interferon drug, such as interferon or pegylated interferon). In certain embodiments, the nanobody or antigen-binding fragment thereof or polypeptide construct of the present invention is used in combination with an additional antiviral agent to prevent and/or treat a disease associated with hepatitis B virus infection. The nanobody or antigen-binding fragment thereof or polypeptide construct of the present invention can be administered simultaneously, separately or continuously with such antiviral agent. Such antiviral reagent includes, but is not limited to, interferon drug, ribavirin, adamantane, IL-2, L-12, etc.

The pharmaceutical composition of the present invention may comprise a "therapeutically effective amount" or a "prophylactically effective amount" of the nanobody or antigen-binding fragment thereof or polypeptide construct of the present invention. "Prophylactically effective amount" refers to an amount sufficient to prevent, arrest, or delay the occurrence of a disease (e.g., HBV infection or a disease associated with HBV infection). "Therapeutically effective amount" refers to an amount sufficient to cure or at least partially prevent a disease and its complications in a patient who is already suffering from the disease. The therapeutically effective amount of the nanobody or antigen-binding fragment thereof or polypeptide construct of the present invention may vary depending on factors such as: the severity of the disease to be treated, the overall state of the patient's own immune system, the patient's general condition such as age, weight and gender, mode of administration, and other treatments administered at the same time, etc.

Dosage regimens can be adjusted to achieve the best desired response (e.g., therapeutic or preventive response). For example, a single dose may be administered, or multiple doses may be administered within a period of time, or the dose can be proportionally reduced or increased as indicated by the exigencies of the therapeutic situation.

### Detection use of antibody

The nanobody or antigen-binding fragment thereof or polypeptide construct or conjugate of the present invention is capable of specifically binding to HBsAg and thus can be used to detect the presence or level of HBsAg in a sample.

Therefore, in another aspect, the present invention provides a method of detecting the presence or level of HBsAg protein in a sample, which comprises: using the nanobody or antigen-binding fragment thereof or polypeptide construct or conjugate of the present invention. In some embodiments, the nanobody or antigen-binding fragments or polypeptide constructs thereof of the present invention further comprise a detectable label. In other embodiments, the method further comprises using a second antibody carrying a detectable label to detect the nanobody or antigen-binding fragment thereof or polypeptide construct of the present invention. The method may be used for diagnostic purposes, or for non-diagnostic purposes (e.g., the sample is a cell sample rather than a sample from a patient).

In certain embodiments, the method comprises: (1) contacting the sample with the nanobody or antigen-binding fragment thereof or polypeptide construct or conjugate of the present invention; (2) detecting the formation of an antigen-antibody complex or detecting the amount of the complex. The formation of the complex indicates the presence of HBsAg protein and/or HBV.

In another aspect, the present invention provides a method for diagnosing whether a subject is infected with HBV, comprising: using the nanobody or antigen-binding fragment thereof or polypeptide construct or conjugate of the present invention to detect the presence of HBsAg protein in a sample from the subject. In some embodiments, the nanobody or antigen-binding fragment thereof or polypeptide construct of the present invention further comprise a detectable label. In other embodiments, the method further comprises using a second antibody carrying a detectable label to detect the nanobody or antigen-binding fragment thereof or polypeptide construct of the present invention.

In another aspect, there is provided a use of the nanobody or antigen-binding fragment thereof or polypeptide construct or conjugate of the present invention in the manufacture of a detection reagent for detecting the presence or level of HBsAg protein in a sample, or for diagnosing whether a subject is infected with HBV.

In any of the above aspects, the HBV involved in the nanobody or antigen-binding fragment thereof or polypeptide construct or conjugate of the present invention comprises but is not limited to genotype A, B, C, D, E, F, G, H, I, J.

### Antibody recognizing epitope peptide and use thereof

In another aspect, the present invention provides an antibody or antigen-binding fragment thereof capable of specifically binding to the epitope peptide or variant thereof or epitope contained therein, recombinant protein, multimer or particle of the present invention.

In certain embodiments, the antibody or antigen-binding fragment thereof competes with the above-mentioned nanobody (e.g., 125s) provided by the present invention for binding to HBsAg protein. The competitive binding refers to the ability to block at least 50%, preferably at least 60%, preferably at least 70%, preferably at least 80%, preferably at least 90%, preferably at least 95% or preferably at least 99% of the binding of the nanobody (e.g., 125s) provided by the present invention to HBsAg protein. The competitive binding can be determined by competitive binding assay.

Competitive binding assay is well known to those skilled in the art. Competitive binding assay is an immunological assay that detects and quantifies an unknown substance through its ability to inhibit the binding of a labeled known antigen to specific antibody thereof, and is also called competitive inhibition assay. An exemplary method comprises: an antigen is first pre-coated on a microplate, then a serially diluted unlabeled antibody to be tested and a labeled known monoclonal antibody (e.g., the nanobody provided by the present invention) with a specific concentration are added together to the above pre-coated microplate for incubation, and then the amount of the known antibody bound to the microplate under the condition of different dilution of the antibody to be tested is determined after washing. The greater the ability of the antibody to be tested to compete with the known antibody for binding to the antigen, the less the ability of the known antibody to bind to the antigen, and the less the amount of the known antibody to bind to the microplate. For example, an antigen is pre-coated on a 96-well microplate, and a radioactive immunoassay, enzyme immunoassay such as ELISA, or fluorescent immunoassay is used to determine the ability of a monoclonal antibody to be tested to block a labeled known monoclonal antibody.

In certain embodiments, the antibody or antigen-binding fragment thereof is selected from Fab, Fab', F(ab')₂, Fd, Fv, dAb, complementarity determining region fragment, single-chain antibody (e.g., scFv), nanoantibody, humanized antibody, chimeric antibody or bispecific or multispecific antibody.

In another aspect, the present invention provides a method for producing an antibody that specifically binds to the epitope peptide or variant thereof of the present invention or an epitope contained therein, the method comprising using the epitope peptide or variant thereof, recombinant protein, multimer, particle, or polynucleotide encoding the epitope peptide or variant thereof or recombinant protein as described in the present invention.

In certain embodiments, the method comprises: immunizing an animal with the epitope peptide or variant thereof, recombinant protein, multimer, particle, or polynucleotide; and screening and obtaining a target antibody from the immunized animal.

In certain embodiments, the method further comprises a humanization process.

In certain embodiments, the humanization process comprises grafting CDR sequences of the antibody produced from the animal into FR framework regions of a human antibody.

In certain embodiments, the humanization process comprises using a transgenic animal (e.g., a transgenic mouse) that is not capable of producing endogenous immunoglobulins and is capable of producing a complete human antibody repertoire upon immunization.

In certain embodiments, the method comprises using a display technology. In certain embodiments, the display technology is selected from the group consisting of phage display, bacterial display, yeast display, or ribosome display. In certain embodiments, the library used in the display technology is designed to have sequences derived from human antibody.

In certain embodiments, the method further comprises using the epitope peptide or variant thereof, recombinant protein, multimer, or particle of the present invention to screen for an antibody that specifically binds to the target epitope.

In another aspect, the present invention further provides an isolated nucleic acid molecule, vector or host cell comprising a nucleotide sequence encoding the above-described antibody or antigen-binding fragment thereof. In another aspect, the present invention provides a pharmaceutical composition comprising the above-mentioned antibody or antigen-binding fragment thereof and a pharmaceutically acceptable carrier and/or excipient.

In another aspect, the present invention provides a method for preventing or treating HBV infection or a disease associated with HBV infection (e.g., hepatitis B) in a subject (e.g., a human), for neutralizing the virulence of HBV in a subject (e.g., a human), for reducing the serum levels of HBV DNA and/or HBsAg in a subject (e.g., a human), and/or for activating an humoral immune response against HBV in a subject (e.g., a subject with chronic HBV infection or a chronic hepatitis B patient), the method comprising administering to the subject in need thereof an effective amount of the above-mentioned antibody or antigen-binding fragment thereof, or the pharmaceutical composition containing the same. The present invention further relates to a use of the above-mentioned antibody or antigen-binding fragment thereof or the pharmaceutical composition containing the same in the manufacture of a medicament for preventing and/or treating HBV infection or a disease related to HBV infection (e.g., hepatitis B) in a subject (e.g., a human), for neutralizing the virulence of HBV in vitro or in a subject (e.g., a human), for reducing the serum levels of HBV DNA and/or HBsAg in a subject (e.g., a human), and/or, for activating an humoral immune response against HBV in a subject (e.g., a subject with chronic HBV infection or a chronic hepatitis B patient).

### Definition of Terms

In the present invention, unless otherwise stated, scientific and technical terms used herein have the meanings commonly understood by those skilled in the art. Moreover, the virology, biochemistry, and immunology laboratory procedures used herein are routine procedures widely used in the corresponding fields. Meanwhile, in order to better understand the present invention, definitions and explanations of relevant terms are provided below.

As used herein, the term "HBsAg" refers to the main surface antigen protein of hepatitis B virus (HBV), which is well known to those skilled in the art (see, for example, NCBI GENBANK database accession number: ADC29595.1).

As used herein, when referring to the amino acid sequence of HBsAg, it is described using the sequence as set forth in SEQ ID NO: 36. For example, the expression "amino acid residues 157 to 174 of HBsAg" refers to amino acid residues 157 to 174 of the polypeptide as set forth in SEQ ID NO: 36. However, those skilled in the art understand that in the amino acid sequence of HBsAg, mutations or variations (including, but not limited to, substitutions, deletions and/or additions, such as different genotypes or gene subtypes of HBsAg) may occur naturally or be introduced artificially without affecting its biological functions. Therefore, in the present invention, the term "HBsAg" shall include all such sequences, including, for example, the sequence as set forth in SEQ ID NO: 36 and natural or artificial variants thereof. Moreover, when describing a sequence fragment of HBsAg, it comprises not only sequence fragments of SEQ ID NO: 36, but also the corresponding sequence fragments in its natural or artificial variants. For example, the expression "amino acid residues 157 to 174 of HBsAg" comprises amino acid residues 157 to 174 of SEQ ID NO: 36, and corresponding fragments in its (natural or artificial) variants. According to the present invention, the expression "corresponding sequence fragment" or "corresponding fragment" refers to a fragment located at equivalent positions in sequences to be compared when the sequences are optimally aligned, that is when the sequences are aligned to obtain the highest percent identity.

As used herein, the term "epitope" refers to an antigen portion capable of being recognized and specifically bound by a particular antibody. When the antigen is a polypeptide, the epitope can be formed by consecutive amino acids or non-consecutive amino acids brought together by the tertiary folding of the protein, called linear epitope or conformational epitope, respectively. Epitopes formed by consecutive amino acids are typically retained upon protein denaturation, whereas epitopes formed by tertiary folding are typically lost upon protein denaturation. Epitopes generally comprise at least 3, 4, 5, 6, 7, 8, 9, 10, 11, 12, 13, 14 or 15 consecutive or non-consecutive amino acids in a unique spatial conformation.

As used herein, the term "epitope peptide" refers to a peptide segment on an antigen capable of functioning as an epitope. In some cases, an epitope peptide alone is capable of being specifically recognized/bound by an antibody directed against that epitope. In other cases, it may be necessary to fuse the epitope peptide to a carrier protein so that the epitope peptide can be recognized by a specific antibody. As used herein, the term "carrier protein" refers to a protein that can act as a carrier for an epitope peptide, that is, it allows the insertion of the epitope peptide at a specific position (e.g., within the protein, at N-terminal or C-terminal), so that the epitope peptide can be presented and recognized by the antibody or immune system. Optionally, the carrier protein is capable of forming a multimer or self-assembling to form a particle. Optionally, a linker can be used between the epitope peptide and the carrier protein to facilitate folding of each.

As used herein, the term "protein-protein binding pair" includes two proteins (e.g., a first member (e.g., a first polypeptide) and a second cognate member (e.g., a second polypeptide)) that interact to form a covalent isopeptide bond bond under conditions that enable or facilitate isopeptide bond formation, wherein the term "cognate" refers to components that function together, i.e. to react together to form an isopeptide bond. Thus, two proteins that react together efficiently to form an isopeptide bond under conditions that enable or facilitate isopeptide bond formation can also be referred to as being a "complementary" pair of peptide linkers. Specific binding pairs capable of interacting to form a covalent isopeptide bond are reviewed in Veggiani G, Zakeri B, Howarth M. Superglue from bacteria: unbreakable bridges for protein nanotechnology. Trends Biotechnol. 2014;32(10):506-512., and non-limiting examples include, SpyTag: SpyCatcher, SpyTag002: SpyCatcher002, SpyTag:KTag, isopeptag:pilinC, SnoopTag: SnoopCatcher, etc.

The term "isopeptide bond" refers to an amide bond between a carboxyl or carboxamide group and an amino group at least one of which is not derived from a protein main chain or alternatively viewed is not part of the protein backbone. An isopeptide bond may form within a single protein or may occur between two peptides or a peptide and a protein. Thus, an isopeptide bond may form intramolecularly within a single protein or intermolecularly i.e. between two peptide/protein molecules, e.g. between two peptide linkers. Typically, an isopeptide bond may occur between a lysine residue and an asparagine, aspartic acid, glutamine, or glutamic acid residue or the terminal carboxyl group of the protein or peptide chain or may occur between the alpha-amino terminus of the protein or peptide chain and an asparagine, aspartic acid, glutamine or glutamic acid.

The SpyTag: Spy Catch er system is described in U.S. Patent No. 9,547,003 and Zakeri et al. Peptide tag forming a rapid covalent bond to a protein, through engineering a bacterial adhesin. Proc Natl Acad Sci USA. 2012;109(12):E690-E697 (which is incorporated herein by reference in its entirety), and is derived from the CnaB2 domain of *Streptococcus pyogenes* fibronectin binding protein FbaB. Another specific binding pair derived from CnaB2 domain is SpyTag:KTag, which forms an isopeptide bond in the presence of SpyLigase, see, Fierer JO, Veggiani G, Howarth M. SpyLigase peptide-peptide ligation multimerizes affibodies to enhance magnetic cancer cell capture. Proc Natl Acad Sci USA. 2014;111(13):E1176-E1181, which is incorporated herein by reference in its entirety. The SpyTag002:SpyCatcher002 system is described in Keeble AH et al. Evolving Accelerated Amidation by SpyTag/SpyCatcher to Analyze Membrane Dynamics. Angew Chem Int Ed Engl. 2017;56(52):16521-16525, which is incorporated herein by reference in its entirety.

The SnoopTag: SnoopCatcher system is described in Veggiani G, Nakamura T, Brenner MD, et al. Programmable polyproteams built using twin peptide superglues. Proc Natl Acad Sci USA. 2016; 113(5):1202-1207, which is incorporated herein by reference in its entirety. The D4 Ig-like domain of RrgA (which adheres to *Streptococcus pneumoniae*) is separated to form SnoopTag and SnoopCatcher.

The isopeptag:pilin-C specific binding pair is derived from the major pilin protein Spy0128 from *Streptococcus pyogenes,* see Zakeri B, Howarth M. Spontaneous intermolecular amide bond formation between side chains for irreversible peptide targeting. J Am Chem Soc. 2010;132(13):4526-4527., which is incorporated herein by reference in its entirety.

In this article, the term "antibody" is used in its broadest sense. "Antibody" includes basic four-chain antibody, heavy chain antibody, nanobody or single domain antibody, monoclonal antibody, polyclonal antibody, bispecific or multispecific antibody, etc., as well as antibody fragment with the required biological activity (e.g., antigen-binding fragment). A basic four-chain antibody usually refers to an immunoglobulin molecule composed of two pairs of polypeptide chains, each pair having a light chain (LC) and a heavy chain (HC). Antibody light chains can be classified into κ (kappa) and λ (lambda) light chains. Heavy chains can be classified as µ, δ, γ, α, or ε, and define the antibody's isotypes as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin with host tissues or factors, including various cells of the immune system (e.g., effector cells) and the first component of classical complement system (C1q). The VH and VL regions can also be subdivided into regions of high variability called complementarity determining regions (CDRs), interspersed with more conservative regions called framework regions (FRs). Each VH and VL consists of 3 CDRs and 4 FRs arranged from the amino terminal to the carboxyl terminal in the following order: FR1, CDR1, FR2, CDR2, FR3, CDR3, FR4. The variable regions (VH and VL) of each heavy chain/light chain pair respectively form the antigen-binding site. The assignment of amino acids to each region or domain can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883. The term "antibody" is not limited to any particular method of producing the antibody. The antibody may be of different isotypes, for example, IgG (e.g., IgG1, IgG2, IgG3 or IgG4 subtypes), IgA1, IgA2, IgD, IgE or IgM antibody.

As used herein, the term "complementarity determining region" or "CDR" refers to the amino acid residues in the variable region of an antibody that are responsible for antigen binding. In a typical four-chain antibody, there are three CDRs in each variable region of the heavy chain and light chain, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883) or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (e.g., see, Lefranc et al., Dev. Comparat. Immunol. 27:55-77, 2003).

As used herein, the term "framework region" or "FR" residues refers to those amino acid residues in an antibody variable region other than the CDR residues as defined above.

As used herein, the term "antigen-binding fragment" of antibody refers to a polypeptide comprising a fragment of a full-length antibody that retains the ability to specifically bind to the same antigen to which the full-length antibody binds, and/or competes with the full-length antibody for specific binding to the antigen, which is also called an "antigen-binding moiety." See, generally, Fundamental Immunology, Ch. 7 (Paul, W., ed., 2nd ed., Raven Press, N.Y. (1989), which is incorporated herein by reference in its entirety for all purposes. The antigen-binding fragment of antibody can be produced by recombinant DNA technology or by enzymatic or chemical cleavage of an intact antibody.

As used herein, the term "camel antibody" refers to an antibody against an antigen produced by an animal of the family Camelidae (including Camel, Alpaca, and L.glama) that has been immunized or infected with the antigen. It is known to those skilled in the art that among the antibodies produced by camelid animals, there is a "Camelid heavy-chain antibodies (HCAb)" that lacks light chain, and thus, this antibody only comprises one variable domain of heavy chain of HCAb (VHH) and two conventional CH2 and CH3 regions, and the VHH region as cloned and expressed alone has good structural stability and antigen-binding activity. VHH is currently known to be the smallest unit capable of binding to antigen.

As used herein, the term "nanobody" is also known as "single domain antibody (sdAb)", which are used interchangeably, and refer to an antibody fragment composed of a single variable domain (e.g., heavy chain variable region) in the antibody, and usually derived from the variable regions of heavy chain antibody (e.g., camelid antibody or shark antibody). Typically, nanobody is composed of 4 framework regions and 3 complementarity determining regions, and has the structure of FR1-CDR1-FR2-CDR2-FR3-CDR3-FR4. Nanobody can be truncated at the N- or C-terminal so that it contains only part of FR1 and/or FR4, or lacks one or two of the framework regions, as long as it substantially retains the antigen binding ability and specificity. Nanobody combines the advantages of monoclonal antibody and small molecule drug. It can penetrate the blood-brain barrier, can be easily engineered, developed and stably produced, thereby reducing the difficulty and cost of production.

As used herein, the term "Fc domain" or "Fc region" refers to a portion of a heavy chain constant region comprising CH2 and CH3. The Fc fragment of an antibody has many different functions but does not participate in antigen binding. "Effector functions" mediated by the Fc region include Fc receptor binding; Clq binding and complement-dependent cytotoxicity (CDC); antibody-dependent cell-mediated cytotoxicity (ADCC); phagocytosis effect; downregulation on cell surface receptor (e.g., B-cell receptor); and B-cell activation, etc. In some embodiments, the Fc region comprises hinge, CH2, and CH3. When the Fc region comprises a hinge, the hinge mediates dimerization between the two Fc-containing polypeptides. The Fc region can be of any isotype of antibody heavy chain constant region, such as IgGl, IgG2, IgG3 or IgG4.

The Fc domain may include either a native Fc region or a variant Fc region. Native Fc region comprises an amino acid sequence that is consistent with the amino acid sequence of Fc region found in nature, for example, a human Fc region with native sequence comprises human IgG1 Fc region (non-A and A allotypes) with native sequence; human IgG2 Fc region with native sequence; human IgG3 Fc region with native sequence; human IgG4 Fc region with native sequence, and naturally occurring variants thereof. A variant Fc region comprises an amino acid sequence that differs from the amino acid sequence of a native sequence Fc region by at least one amino acid modification. In some embodiments, a variant Fc region may possess altered effector functions (e.g., Fc receptor binding, antibody glycosylation, number of cysteine residues, effector cell function, or complement function) as compared to a native Fc region.

As used herein, the term "humanized antibody" refers to a non-human antibody that has been genetically engineered, the amino acid sequence of which has been modified to increase sequence homology to that of a human antibody. Generally speaking, all or part of CDR regions of a humanized antibody are derived from a non-human antibody (donor antibody), and all or part of non-CDR regions (e.g., variable region FR and/or constant region) are derived from a human immunoglobulin (receptor antibody). In certain embodiments, the CDR regions of humanized antibody are derived from a non-human antibody (donor antibody), and all or part of non-CDR regions (e.g., variable region FR and/or constant region) are derived from a human immunoglobulin (receptor antibody). Humanized antibodies generally retain the expected properties of the donor antibody, including but not limited to, antigen specificity, affinity, reactivity, etc. In the present application, the donor antibody may be a camel antibody with desired properties (e.g., antigen specificity, affinity, reactivity, etc.). To prepare a humanized antibody, the CDR regions of an immunized animal can be inserted into a human framework sequence using the methods known in the art. In the context of nanobody, a humanized antibody may refer to a humanized VHH, that is, a VHH in which one or more framework regions have been substantially replaced by human framework regions. In some cases, certain framework regions (FRs) of human immunoglobulin are replaced by corresponding non-human residues. Additionally, a humanized VHH may contain residues that are not found in either the original VHH or the human framework sequence, which are included to further improve and optimize the performance of the VHH or VHH-containing polypeptide.

As used herein, the term "degree of humanization" refers to an indicator used to express the number of non-human amino acid residues in a humanized antibody. The degree of humanization of a humanized antibody can be calculated, for example, as follows: degree of humanization = (number of amino acids in FR region - number of non-human amino acids retained in FR region)/number of amino acids in FR region × 100%.

As used herein, the term "germline antibody gene" or "germline antibody gene segment" refers to a sequence present in the genome of an organism encoding immunoglobulin, which has not undergone a maturation process that can lead to genetic rearrangements and mutations for expression of a particular immunoglobulin. In the present invention, the expression "heavy chain germline gene" refers to an germline antibody gene or gene fragment encoding an immunoglobulin heavy chain, which includes V gene (variable), D gene (diversity), J gene (joining) and C gene (constant); similarly, the expression "light chain germline gene" refers to an germline antibody gene or gene fragment encoding an immunoglobulin light chain, which includes V gene (variable), J gene (joining), and C gene (constant). In the present invention, the amino acid sequence encoded by the germline antibody gene or the germline antibody gene fragment is also referred to as "germline sequence". The germline antibody gene or germline antibody gene fragment and their corresponding germline sequences are well known to those skilled in the art and can be obtained or queried from professional databases (e.g., IMGT, unswag, NCBI or VBASE2).

As used herein, the term "specific binding" refers to a non-random binding reaction between two molecules, such as the reaction between an antibody and an antigen against which it is directed. The strength or affinity of a specific binding interaction can be expressed by the equilibrium dissociation constant (K_{D}) of the interaction. In the present invention, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen.

The specific binding properties between two molecules can be determined using methods known in the art. One approach involves measuring the rates at which antigen binding site/antigen complex form and dissociate. Both the "association rate constant" (ka or kon) and the "dissociation rate constant" (kdis or koff) can be calculated from concentrations and the actual rates of association and dissociation (see, Malmqvist M, Nature, 1993, 361 :186-187). The ratio kdis/kon is equal to the dissociation constant K_{D} (see, Davies et al., Annual Rev Biochem, 1990; 59:439-473). The values of K_{D}, kon and kdis can be measured using any valid method, such as surface plasmon resonance (SPR) in Biacore to measure the dissociation constant, or bioluminescence interferometry or Kinexa to measure the dissociation constant.

As used herein, "neutralizing antibody" refers to an antibody or antigen-binding fragment thereof that is capable of significantly reducing or completely inhibiting the virulence (e.g., the ability to infect cells) of a target virus. Generally speaking, neutralizing antibodies are able to recognize and bind to the target virus and prevent the target virus from entering/infecting the subject's cells. The antibody of the present invention is a neutralizing antibody.

However, it should be understood that in the present application, the ability of an antibody to neutralize a virus is not directly equivalent to the ability of the antibody to eliminate the virus. As used herein, "neutralizing virus" means that the virulence of a target virus is neutralized (i.e. the virulence of a target virus is significantly reduced or completely inhibited) by inhibiting the target virus from entering/infecting the cell of a subject. As used herein, "eliminating virus" means that a target virus (no matter it infects a cell or not) is eliminated from an organism, and therefore the organism turns toward the state before infection by the virus (e.g. the serological test result of virus turns negative). Therefore, in general, neutralizing antibodies do not necessarily have virus-eliminating ability. However, in the present application, the inventor surprisingly found that the antibodies according to the invention can not only neutralize HBV, but also clear virus (i.e. can clear HBV DNA and/or HBsAg *in vivo,* clear HBV and HBV-infected cells *in vivo*), and therefore have important clinical value.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); phages such as λ phage or M13 phage, and animal viruses, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may contain a variety of expression control elements, including, but not limited to, promoter sequence, transcription initiation sequence, enhancer sequence, selection element and reporter gene. In addition, the vector may also contain an origin of replication site.

As used herein, the term "host cell" refers to a cell that can be used to introduce a vector, which comprises, but is not limited to, prokaryotic cells such as *E. coli* or *Bacillus subtilis,* fungal cells such as yeast cells or *Aspergillus,* insect cells such as *S2 Drosophila* cells or Sf9, or animal cells such as fibroblasts, CHO cells, COS cells, NSO cells, HeLa cells, BHK cells, HEK 293 cells or human cells.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

As used herein, the term "conservative substitution" refers to an amino acid substitution in an amino acid sequence that does not adversely affect or alter the expected properties of the protein/polypeptide comprising the amino acid sequence. For example, conservative substitution can be introduced by standard techniques known in the art, such as site-directed mutagenesis and PCR-mediated mutagenesis. Conservative amino acid substitution comprises substitution with an amino acid residue having a similar side chain, for example, substitution with an amino acid residue that is physically or functionally similar to the corresponding amino acid residue (e.g., having similar size, shape, charge, chemical properties, including ability to form covalent bond or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to substitute the corresponding amino acid residue with another amino acid residue from the same side chain family. In addition, amino acid residues can be divided into categories defined by optional physical and functional properties, for example, alcohol-containing residues (S and T), aliphatic residues (I, L, V, and M), cycloalkenyl-related residues (F, H, W, and Y), hydrophobic residues (A, C, F, G, H, I, L, M, R, T, V, W, and Y), negatively charged residues (D and E), polar residues (C, D, E, H, K, N, Q, R, S and T), positively charged residues (H, K and R), small residues (A, C, D, G, N, P, S, T and V), very small residues (A, G and S), residues involved in corner formation (A, C, D, E, G, H, K, N, Q, R, S, P and T), flexible residues (Q, T, K, S, G, P, D, E and R). Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which are incorporated herein by reference).

The twenty conventional amino acids involved herein have been written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with a subject and an active ingredient, and they are well known in the art (see, for example, Remington's Pharmaceutical Sciences. Edited by Gennaro AR, 19th ed. Pennsylvania: Mack Publishing Company, 1995), including, but not limited to: pH adjusting agent, surfactant, adjuvant, ionic strength enhancer, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, etc. The agent for maintaining osmotic pressure includes, but is not limited to, sugar, NaCl, and the like. The agent for delaying absorption includes, but is not limited to, monostearate and gelatin.

As used herein, the term "prevention/preventing" refers to a method that is carried out in order to suppress or delay the occurrence of a disease, a disorder or a symptom (such as HBV infection or a disease associated with HBV infection) in a subject. As used herein, the term "treatment/treating" refers to a method that is carried out in order to obtain a beneficial or desired clinical outcome. For the purpose of the invention, the beneficial or desired clinical outcome includes, but is not limited to, easing symptom, narrowing the scope of disease, stabilizing (i.e. not aggravating) the state of disease, delaying or slowing the progress of disease, and alleviating symptoms (either partially or completely), no matter detectable or not detectable. In addition, "treatment" also refers to a prolonged survival period compared to the expected survival period (if no treatment is accepted). In the present application, the antibody of the present invention has the ability to neutralize HBV and thus can be used to prevent/protect an unaffected subject or a cell thereof from infection by HBV. In addition, the antibody of the present invention has the ability to eliminate HBV (i.e., capable of eliminating HBV DNA and/or HBsAg in the body, and eliminating HBV and HBV-infected cells in the body), and thus can be used to treat HBV infection or a disease associated with HBV infection in a subject.

As used herein, the term "subject" refers to a mammal, such as a primate mammal, such as a human.

As used herein, the term "an effective amount" refers to an amount that is sufficient to achieve or at least partially achieve the expected effect. For example, an amount effective for preventing a disease (such as HBV infection or diseases associated with HBV infection) refers to an amount effective for preventing, suppressing, or delaying the occurrence of a disease (such as HBV infection or diseases associated with HBV infection). An effective amount for treating a disease refers to an amount effective for curing or at least partially blocking a disease and its complication in a patient having the disease. The determination of such an effective amount is within the ability of a person skilled in the art. For example, an amount effective for a therapeutic use depends on severity of a disease to be treated, general state of the immune system in a patient, general conditions of a patient, such as age, weight and gender, administration means of drugs, additional therapies used simultaneously, and the like.

### Beneficial effects

It is known in the art that patients with chronic hepatitis B often develop immune depletion (tolerance) against HBV due to high levels of HBsAg in the body, which leads to prolonged infection. The epitope peptide or nanobody of the present invention can activate the regeneration of humoral immune responses against HBV in a subject (e.g., a subject with chronic HBV infection, or a patient with chronic hepatitis B), thereby reducing the serum levels of HBV DNA and/or HBsAg in the subject, and effectively eliminating HBV and HBV-infected cells in the body. Therefore, the epitope peptide or nanobody of the present invention is suitable for preventing and treating HBV infection and a disease related to HBV infection (e.g., hepatitis B), and has broad-spectrum binding activity and broad-spectrum therapeutic activity against multiple genotypes of HBV. The immunotherapy mediated by the epitope peptide or nanobody of the present invention is expected to become a new strategy for CHB patients to combat persistent infection with HBV.

### Brief Description of the Drawings

FIG. 1 shows the binding activity of 125s to HBsAg. The results showed that 125s had good binding activity to HBsAg.

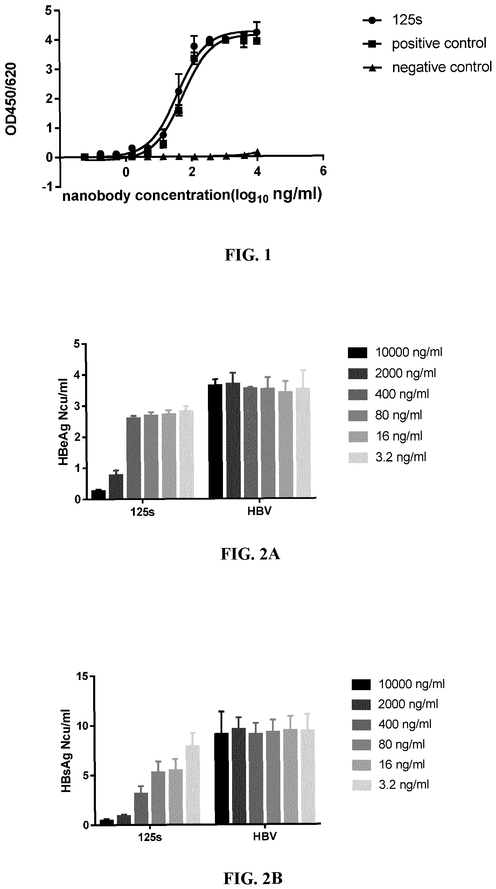

FIG. 2 shows the neutralizing activity of 125s in the HepaRG cell model. In FIGs. 2A to 2B, "125s" on the left side refers to the experimental group in which the antibody to be tested is added, and "HBV" on the right side refers to the control group in which the antibody to be tested is replaced with PBS. The control group reflects the secretion level of HbeAg (FIG. 2A) or HBsAg (FIG. 2B) in HepaAD38 cells without treatment with neutralizing antibody. FIG. 2A shows that as the 125s antibody concentration increases, the HBeAg content in the cell supernatant decreases significantly; FIG. 2B shows that as the 125s antibody concentration increases, the HBsAg content in the cell supernatant decreases significantly; suggesting that 125s has neutralizing activity.
FIG. 3 shows that 125s has binding activity to HBsAg particles of nine HBV genotypes (A to H; J), suggesting that 125s has broad-spectrum binding activity.
FIG. 4 shows the therapeutic effect of 125s in the HBV-AAV mouse model, wherein:
   FIG. 4A shows that after 125s single-injection treatment at a dose of 10mg/kg in the mouse model of AAV-adw serotype, the HBsAg titer in the serum was less than 100IU/ml and could be maintained for at least 7 days;
   FIG. 4B shows that after 125s single-injection treatment at a dose of 10 mg/kg in the mouse model of AAV-ayw serotype, the HBsAg titer in the serum was less than 100 IU/ml and could be maintained for at least 7 days. The therapeutic effect in HBV-AAV mice suggests that 125s has long-lasting HBsAg inhibition and broad-spectrum therapeutic activity.
FIG. 5 shows that after 125s single-injection treatment at a dose of 10 mg/kg in the HBV transgenic mouse model, the HBsAg titer in the serum rapidly decreased to less than 100IU/ml, and the HBsAg suppression state could be maintained for 7 days, which slowly returned to the baseline level from day 11 to day 14 after the treatment. The therapeutic effect in HBV transgenic mice suggests that 125s has long-lasting HBsAg inhibitory activity.
FIG. 6 shows the binding of 125s to denatured HBsAg.

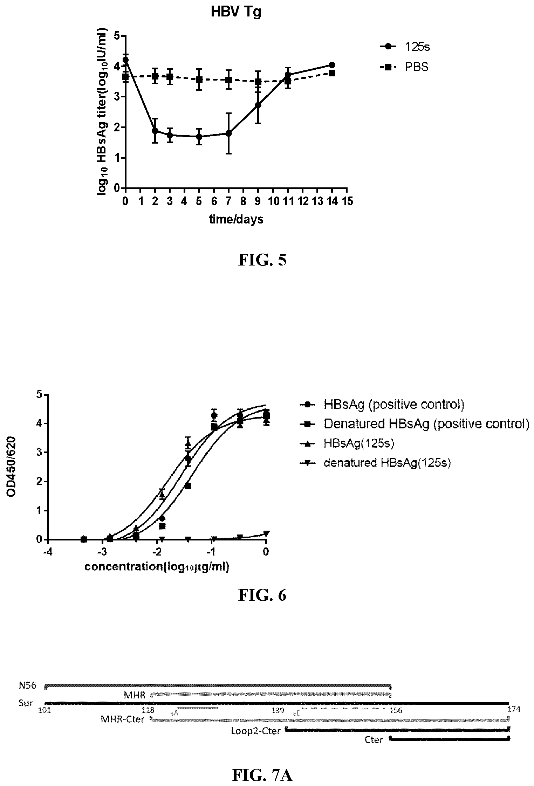

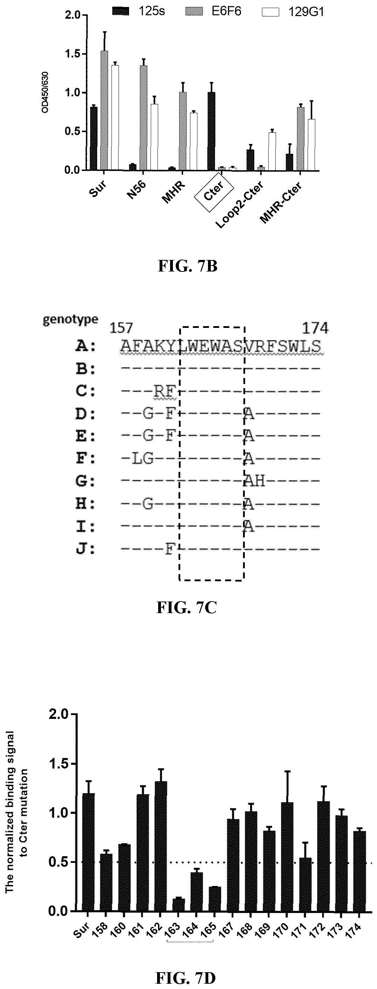

FIG. 7 shows the recognition epitope of 125s on HBsAg. FIG. 7A shows a schematic diagram of the display format of the HBsAg extracellular segment fusion protein, which comprises the extracellular segment of HBsAg (Surface, referred to as Sur); the N-terminal 56 amino acids of the HBsAg extracellular segment (N56); the main hydrophilic region (MHR) of the HBsAg extracellular segment; the C-terminal 18 amino acids of the HBsAg extracellular segment (Cter); the C-terminal 36 amino acids of the HBsAg extracellular segment (Loop2-Cter); the MHR and Cter portion of the HBsAg extracellular segment (MHR-Cter). FIG. 7B shows the binding activity of 125s to 6 HBsAg extracellular segment fusion proteins (Sur; N56; MHR; Cter; Loop2-Cter; MHR-Cter), in which the results show that 125s has obvious binding reactivity to the complete extracellular segment of HBsAg (Sur) and to the C-terminal 18 amino acids of the extracellular segment of HBsAg (Cter), suggesting that the main binding site of 125s is located at the C-terminal of HBsAg. FIG. 7C shows the conservation analysis of the Cter region (aa157 to 174) of 10 HBV genotypes (A to J). FIG. 7D shows the binding activity of 125s to the HBsAg extracellular segment fusion protein with an alanine single point mutation in positions 158 to 174. The results show that when the single point mutation occurred at positions 158, 160, 163 to 165, and 171, the binding activity of 125s to the HBsAg extracellular segment fusion protein was significantly reduced, suggesting that the main binding epitope of 125s is related to positions 158, 160, 163 to 165, and 171.
FIG. 8 shows that the humanized antibody (h125s-26) molecule of 125s had a binding activity to HBsAg comparable to that of the parental 125s nanobody, indicating that the humanized modification of the humanized antibody (h125s-26) molecule of 125s was successful.
FIG. 9 shows that after the single injection of the humanized nanobody molecule h125s-26, HBsAg in mouse serum was rapidly eliminated, suggesting that the humanized molecule h125s-26 of 125s nanobody has in vivo therapeutic activity.
FIG. 10 shows that the Cter-Fc fusion protein has immunogenicity.
FIG. 11 shows that the purified Spytag-murine ferritin exhibited a nanoparticle structure with uniform size, and stable structure and morphology.
FIG. 12 shows that the Spytag-murine ferritin and Spycatcher-Cter could be assembled to form a particle.

### Sequence information

A description of the sequences involved in the present application is provided in the table below.

**Table 1: Sequence information**

| SEQ ID NO: | Description | Sequence |
|---|---|---|
| 1 | 125s VHH-CDR1 | GGTISAYA |
| 2 | 125s VHH-CDR2 | ISWTTSSA |
| 3 | 125s VHH-CDR3 | NDRVSDTVGFGS |
| 4 | 125s VHH-FR1 | QLQLVESGGGLVQAGGSLRLSCAAS |
| 5 | 125s VHH-FR2 | MGWFRQAPGKDREFVAG |
| 6 | 125s VHH-FR3 | YYTDSVKGRFTISRDFAKNTVYLQMNSLKPEDTA VYYC |
| 7 | 125s VHH-FR4 | WGQGTQVTVSS |
| 8 | 125s VHH | QLQLVESGGGLVQAGGSLRLSCAASGGTISAYAM GWFRQAPGKDREFVAGISWTTSSAYYTDSVKGRF TISRDFAKNTVYLQMNSLKPEDTAVYYCNDRVSD TVGFGSWGQGTQVTVSS |
| 9 | h125s-26 VHH-FR1 | EVQLVESGGGLVQPGGSLRLSCAAS |
| 10 | h125s-26 VHH-FR2 | MGWFRQAPGKDREFVAG |
| 11 | h125s-26 VHH-FR3 | YYTDSVKGRFTISRDFAKNTVYLQMNSLKPEDTA VYYC |
| 12 | h125s-26 VHH-FR4 | WGQGTLVTVSS |
| 13 | h125s-26 VHH | EVQLVESGGGLVQPGGSLRLSCAASGGTISAYAM GWFRQAPGKDREFVAGISWTTSSAYYTDSVKGRF TISRDFAKNTVYLQMNSLKPEDTAVYYCNDRVSD TVGFGSWGQGTLVTVSS |
| 14 | hIgG1-Fc | PKSCDKTHTCPPCPAPELLGGPSVFLFPPKPKDTLM ISRTPEVTCVVVDVSHEDPEVKFNWYVDGVEVHN AKTKPREEQYNSTYRVVSVLTVLHQDWLNGKEY KCKVSNKALPAPIEKTISKAKGQPREPQVYTLPPSR DELTKNQVSLTCLVKGFYPSDIAVEWESNGQPEN NYKTTPPVLDSDGSFFLYSKLTVDKSRWQQGNVF SCSVMHEALHNHYTQKSLSLSPGK |
| 15 | mIgG2-Fc | PRVPITQNPCPPLKECPPCAAPDLLGGPSVFIFPPKI KDVLMISLSPMVTCVVVDVSEDDPDVQISWFVNN VEVHTAQTQTHREDYNSTLRVVSALPIQHQDWMS GKEFKCKVNNRALPSPIEKTISKPRGPVRAPQVYV LPPPAEEMTKKEFSLTCMITGFLPAEIAVDWTSNG RTEQNYKNTATVLDSDGSYFMYSKLRVQKSTWER GSLFACSVVHEGLHNHLTTKTISRSLGK |
| 16 | HBsAg extracellular segment S1 | QGMLPVCPLIPGSSTTSTGPCKTCTTPAQGNSTFPS CCCIKPTDGNCTCIPIPSSWAFAKYLWEWASVRFS WLS |
| 17 | HBsAg extracellular segment S2 | QGMLPVCPLIPGSSTTSTGPCKTCTTPAQGNSTFPS CCCIKPTDGNCTCIPIPSSW |
| 18 | HBsAg extracellular segment S3 | TGPCKTCTTPAQGNSTFPSCCCIKPTDGNCTCIPIPS SW |
| 19 | HBsAg extracellular segment S4 | AFAKYLWEWASVRFSWLS |
| 20 | HBsAg extracellular segment S5 | CIKPTDGNCTCIPIPSSWAFAKYLWEWASVRFSWL S |
| 21 | HBsAg extracellular segment S6 | TGPCKTCTTPAQGNSTFPSCCCIKPTDGNCTCIPIPS SWAFAKYLWEWASVRFSWLS |
| 22 | HBsAg extracellular segment S1 - F158A | QGMLPVCPLIPGSSTTSTGPCKTCTTPAQGNSTFPS CCCIKPTDGNCTCIPIPSSWAAAKYLWEWASVRFS WLS |
| 23 | HBsAg extracellular segment S1 - K160A | QGMLPVCPLIPGSSTTSTGPCKTCTTPAQGNSTFPS CCCIKPTDGNCTCIPIPSSWAFAAYLWEWASVRFS WLS |
| 24 | HBsAg extracellular segment S1 - Y161A | QGMLPVCPLIPGSSTTSTGPCKTCTTPAQGNSTFPS CCCIKPTDGNCTCIPIPSSWAFAKALWEWASVRFS WLS |
| 25 | HBsAg extracellular segment S1 - L162A | QGMLPVCPLIPGSSTTSTGPCKTCTTPAQGNSTFPS CCCIKPTDGNCTCIPIPSSWAFAKYAWEWASVRFS WLS |
| 26 | HBsAg extracellular segment S1 - W163A | QGMLPVCPLIPGSSTTSTGPCKTCTTPAQGNSTFPS CCCIKPTDGNCTCIPIPSSWAFAKYLAEWASVRFS WLS |
| 27 | HBsAg extracellular segment S1 - W165A | QGMLPVCPLIPGSSTTSTGPCKTCTTPAQGNSTFPS CCCIKPTDGNCTCIPIPSSWAFAKYLWEAASVRFS WLS |
| 28 | HBsAg extracellular segment S1 - S167A | QGMLPVCPLIPGSSTTSTGPCKTCTTPAQGNSTFPS CCCIKPTDGNCTCIPIPSSWAFAKYLWEWAAVRFS WLS |
| 29 | HBsAg extracellular segment S1 - V168A | QGMLPVCPLIPGSSTTSTGPCKTCTTPAQGNSTFPS CCCIKPTDGNCTCIPIPSSWAFAKYLWEWASARFS WLS |
| 30 | HBsAg extracellular segment S1 - R169A | QGMLPVCPLIPGSSTTSTGPCKTCTTPAQGNSTFPS CCCIKPTDGNCTCIPIPSSWAFAKYLWEWASVAFS WLS |
| 31 | HBsAg extracellular segment S1 - F170A | QGMLPVCPLIPGSSTTSTGPCKTCTTPAQGNSTFPS CCCIKPTDGNCTCIPIPSSWAFAKYLWEWASVRAS WLS |
| 32 | HBsAg extracellular segment S1 - S171A | QGMLPVCPLIPGSSTTSTGPCKTCTTPAQGNSTFPS CCCIKPTDGNCTCIPIPSSWAFAKYLWEWASVRFA WLS |
| 33 | HBsAg extracellular segment S1 - W172A | QGMLPVCPLIPGSSTTSTGPCKTCTTPAQGNSTFPS CCCIKPTDGNCTCIPIPSSWAFAKYLWEWASVRFS ALS |
| 34 | HBsAg extracellular segment S1 - L173A | QGMLPVCPLIPGSSTTSTGPCKTCTTPAQGNSTFPS CCCIKPTDGNCTCIPIPSSWAFAKYLWEWASVRFS WAS |
| 35 | HBsAg extracellular segment S1 - S174A | QGMLPVCPLIPGSSTTSTGPCKTCTTPAQGNSTFPS CCCIKPTDGNCTCIPIPSSWAFAKYLWEWASVRFS WLA |
| 36 | HBsAg | MENIASGLLGPLLVLQAGFFLLTKILTIPKSLDSWW TSLNFLGGTPVCLGQNSQSQISNHSPTCCPPICPGY RWMCLRRFIIFLCILLLCLIFLLVLLDYQGMLPVCP LIPGSSTTSTGPCKTCTTPAQGNSTFPSCCCIKPTDG NCTCIPIPSSWAFAKYLWEWASVRFSWLSLLVPFV QWFVGLSPTVWLSVIWMMWYWGPSLYNILSPFM PLLPIFCCLWVYS |
| 37 | HBsAg aa163-166 | WEWA |
| 38 | HBsAg aa157-174 (genotype C) | AFARFLWEWASVRFSWLS |
| 39 | HBsAg aa157-174 (genotype D/E) | AFGKFLWEWASARFSWLS |
| 40 | HBsAg aa157-174 (genotype F) | ALGKYLWEWASARFSWLS |
| 41 | HBsAg aa157-174 (genotype G) | AFAKYLWEWASVHFSWLS |
| 42 | HBsAg aa157-174 (genotype H) | AFGKYLWEWASARFSWLS |
| 43 | HBsAg aa157-174 (genotype I) | AFAKYLWEWASARFSWLS |
| 44 | HBsAg aa157-174 (genotype J) | AFAKFLWEWASVRFSWLS |
| 45 | Murine ferritin | MTTASPSQVRQNYHQDAEAAINRQINLELYASYV YLSMSCYFDRDDVALKNFAKYFLHQSHEEREHAE KLMKLQNQRGGRIFLQDIKKPDRDDWESGLNAME CALHLEKSVNQSLLELHKLATDKNDPHLCDFIETY YLSEQVKSIKELGDHVTNLRKMGAPEAGMAEYLF DKHTLGHGDES |
| 46 | SpyTag | AHIVMVDAYKPTKSYY |
| 47 | SpyCatcher full-length sequence | MSYYHHHHHHDYDIPTTENLYFQGAMVDTLSGLS SEQGQSGDMTIEEDSATHIKFSKRDEDGKELAGAT MELRDSSGKTISTWISDGQVKDFYLYPGKYTFVET AAPDGYEVATAITFTVNEQGQVTVNGKATKGDAH I |
| 48 | SpyCatcher truncated sequence | MSYYHHHHHHDYDIPTTENLYFQGDSATHIKFSK RDEDGKELAGATMELRDSSGKTISTWISDGQVKD FYLYPGKYTFVETAAPDGYEVATAITFTVNEQGQ VTVNGKATKGDAHI |
| 49 | SpyTag-murine ferritin | AHIVMVDAYKPTKSYYGGGGSGGGGSGGGGSMT TASPSQVRQNYHQDAEAAINRQINLELYASYVYLS MSCYFDRDDVALKNFAKYFLHQSHEEREHAEKL MKLQNQRGGRIFLQDIKKPDRDDWESGLNAMECA LHLEKSVNQSLLELHKLATDKNDPHLCDFIETYYL SEQVKSIKELGDHVTNLRKMGAPEAGMAEYLFDK HTLGHGDES |
| 50 | SpyCatcher-Cter | MSYYHHHHHHDYDIPTTENLYFQGDSATHIKFSK RDEDGKELAGATMELRDSSGKTISTWISDGQVKD FYLYPGKYTFVETAAPDGYEVATAITFTVNEQGQ VTVNGKATKGDAHIGGGGSGGGGSGGGGSAFAK YLWEWASVRFSWLS |
| 51 | General formula of aa157-174 | AX₁X₂X₃X₄X₅WEWAX₆X₇X₈X₉SX₁₀X₁₁X₁₂ |

### Examples

The embodiments of the present invention will be described in detail below with reference to examples. Those skilled in the art will understand that the following examples are only used to illustrate the present invention and should not be regarded as limiting the scope of the present invention. For the specific techniques or conditions that are not specified in the examples, the techniques or conditions described in literature in the field (e.g., refer to J. Sambrook et al., "Molecular Cloning Experimental Guide", translated by Huang Peitang et al., third edition, Science Press), or the product instructions shall be followed. For the reagents or instruments where the manufacturer is not indicated, they are all conventional products that can be purchased commercially.

### Example 1: Preparation of anti-HBsAg nanobody

### 1.1 Preparation of immunogen

The immunogen was CHO-expressed recombinant hepatitis B virus surface antigen main protein (HBsAg, genotype B, purchased from Beijing Wantai Biopharmaceutical Co., Ltd.). The recombinant protein was diluted to 0.4 mg/ml and mixed with an equal volume of Freund's adjuvant for complete emulsification. Freund's complete adjuvant was used for the initial immunization, and Freund's incomplete adjuvant was used for subsequent booster immunizations.

### 1.2 Construction and screening of nanobody phage library

The above immunogen was used to immunize healthy adult alpacas for a total of 4 times. After the immunization was completed, the alpaca peripheral blood was extracted, total RNA was isolated, and the VHH gene was obtained by reverse transcription and PCR amplification. Then the VHH gene was cloned into the phage vector Pcgmt (preserved in the laboratory), transformed into ER2738 host cells (purchased from Lucigen) to construct a phage display library. Subsequently, phage display technology was used for library screening, and 96 phage clones were randomly selected for ELISA identification, wherein, the positive monoclone 125s had good binding ability to HBsAg at the phage level. Further, the sequence of 125s was determined using the method described by Kabat et al. The amino acid sequences of the complementarity determining regions CDR1 to CDR3 of the nanobody 125s were set forth in SEQ ID NOs: 1-3, respectively, and the amino acid sequences of its framework regions FR1 to FR4 were set forth in SEQ ID NOs: 4-7, respectively. The amino acid sequence of the VHH domain of the nanobody 125s was set forth in SEQ ID NO: 8.

### 1.3 Eukaryotic expression of anti-HBsAg nanobody

The VHH domain of nanobody 125s was linked to the N-terminal of human IgG1 Fc fragment (SEQ ID NO: 14) or murine IgG2 Fc fragment (SEQ ID NO: 15) to obtain nanobody-Fc fusion proteins 125shIgG1 and 125smIgG2, respectively. Specifically, the VHH domain gene of nanobody 125s was cloned into the ptt5hIgG1 vector (containing the human Fc region as set forth in SEQ ID NO: 14) or the 125smIgG2 vector (containing the murine Fc region as set forth in SEQ ID NO: 15), then the recombinant plasmid and transfection reagent PEI were mixed at 1:2 and transfected into HEK293F cells; the cells were cultured at 37°C and 5% CO₂ for 6 days in a shaking incubator. The cell supernatant was collected, purified with Protein A, measured for concentration by UV spectroscopy, and then aliquoted into 1.5 mL tubes, and stored at -80°C for later use.

### Example 2: Binding activity of nanobody 125s with HBsAg

125shIgG1 was diluted with 20mM PBS buffer for 3-fold gradient dilution with a starting concentration of 1 µg/mL and a total of 12 gradients. Humanized antibody 162 (described in detail in Chinese patent application CN201610879693.5) was used as a positive control, and an irrelevant antibody was used as a negative control. 100 µL of the diluted sample was added to each well of the HBsAg plate and placed in a 37°C incubator for reaction for 60 minutes. The ELISA plate was washed 5 times with PBST washing solution, then 100 µL of HRP-labeled goat anti-human (GAH) IgG reaction solution was added to each well, and placed in a 37°C incubator for reaction for 30 minutes. After completing the enzyme labeling reaction step, the ELISA plate was washed 5 times with PBST washing solution, and 50 µL of TMB chromogenic reagent was added to each well, and placed in a 37°C incubator for reaction for 15 minutes. After completing the color development reaction step, 50 µL of stop solution was added to each well of the ELISA plate, and the OD450/630 value of each well was detected on a microplate reader. The results showed that 125shIgG1 had HBsAg-specific binding activity (FIG. 1).

### Example 3: Determination of neutralizing activity of nanobody 125s

HepaAD38 cells were cells prepared in our laboratory that could controllably express HBV. When it was necessary to expand HepaAD38 cells without expressing HBV, tetracycline could be added to the culture medium to inhibit the transcription and replication of HBV. When it was necessary to express HBV, tetracycline-free medium could be used for culture to initiate HBV transcription and replication. The culture supernatant derived from HepaAD38 cells was contacted with differentiated HepaRG cells, and then the culture supernatant was determined to contain HBV virus indicating that differentiated HepaRG cells were effectively infected. The above-mentioned HepaRG/HBV infection model was used to evaluate the ability of nanobody 125s to neutralize/block HBV infection (100MOI HBV infection titer).

125shIgG1 with a specified concentration was added in advance to the virus solution used to infect cells, and then the virus solution was used to infect HepaRG cells. On the day 7 after infection, the cell culture supernatant was taken, and its HBeAg level and HBsAg level (important indicators of successful HBV infection) were determined. The detection method was as follows:
Anti-HBeAg (or anti-HBsAg) monoclonal antibody was diluted in 20mM PB7.4 to reach a final concentration of 2µg/ml. The diluted anti-HBeAg (or anti-HBsAg) monoclonal antibody was added to a 96-well microplate and incubated overnight at 4°C. The 96-well microplate was washed once with PBST and spin-dried. Then, 200 µL of blocking solution was added to each well. 100 µl/well of the sample to be tested was added and incubated at 37°C for 60 min. The 96-well microplate was washed 5 times with PBST. Horseradish peroxidase-labeled anti-HBeAg (or anti-HBsAg) antibody was added and incubated at 37°C for 30 minutes. The reagents used in the above procedure were purchased from Beijing Wantai Biopharmaceutical Co., Ltd. The 96 microwell plate was washed 5 times with PBST. Chemiluminescence reagent was added to develop color. The values were read using a chemiluminescence microplate reader.

The experimental results were shown in FIGs. 2A to 2B, showing that 125s had good virus neutralizing activity.

### Example 4: Determination of binding activity of nanobody 125s to HBsAg particles of nine HBV genotypes

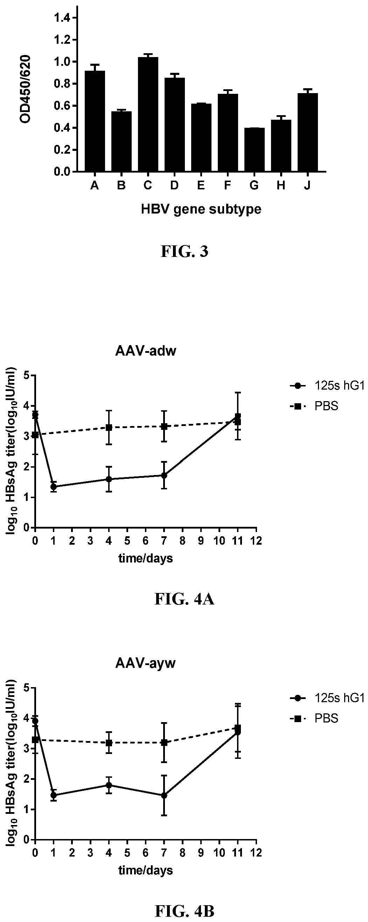

HBsAg particles of a total of 9 HBV genotypes A to H and J prepared in the laboratory were subjected to the detection of binding ability to nanobody 125s by ELISA. The genotypes of HBV could be found in the NCBI taxonomy database (Taxonomy), with the accession number txid10407. The specific detection method was as follows:
HBsAg particles of 9 genotypes were diluted with 20mM PBS buffer to a final concentration of 2µg/mL, added at 100µL per well to Elisa plate, and placed in a 37°C incubator for reaction for 60 minutes. After the Elisa plate was washed once with PBST washing solution, 200 µL of blocking solution was added to each well and incubated at 37°C for 2 hours. After the Elisa plate was washed once with PBST washing solution, 125s nanobody diluted to a final concentration of 0.1 µg/mL was added, 100 uL per well, and placed in a 37°C incubator for reaction for 1 hour. The Elisa plate was washed 5 times with PBST washing solution, and 100 µL of HRP-labeled goat anti-human IgG reaction solution was added to each well, and placed in a 37°C incubator for reaction for 30 minutes. After the enzyme labeling reaction step was completed, the Elisa plate was washed 5 times with PBST washing solution, and 50 µL of TMB chromogenic reagent was added to each well, and placed in a 37°C incubator for reaction for 15 minutes. After the color development reaction step was completed, 50 µL of stop solution was added to each well of the ELISA plate, and the OD450/630 value of each well was detected on a microplate reader. The results showed that 125shIgG1 had broad-spectrum HBsAg-specific binding activity (FIG. 3).

### Example 5: Therapeutic effect of nanobody 125s in HBV-AAV mice of three serotypes

### 5.1 Method for construction of AAV-HBV mouse model

HBV-AAV viruses included HBV-adw serotype (HBV-B type, purchased from Guangzhou Paizhen Biotechnology Co., Ltd.) and HBV-ayw serotype (HBV-D genotype, purchased from Guangzhou Paizhen Biotechnology Co., Ltd.). The above-mentioned viruses were injected into C57BL/6 mice (purchased from Shanghai Slac Experimental Animal Co., Ltd.) via a single injection through the tail vein at a dose of 10E11 GC (genome copies)/mouse, and the modeling period was 30 days.

### 5.2 Method for detection of mouse serum HBsAg

(1) Preparation of reaction plate: Mouse monoclonal antibody HBs-45E9 (Beijing Wantai Biopharmaceutical Co., Ltd.) was diluted to 2 µg/mL with 20mM PB buffer (Na₂HPO₄/NaH₂PO₄ buffer, pH7.4), and 100 µL of coating solution was added to each well of the chemiluminescent plate to perform coating at 2 to 8 °C for 16 to 24 hours, and then at 37°C for 2 hours. The plate was washed once with PBST washing solution and spin-dried. After washing, 200 µL of blocking solution was added to each well to perform blocking at 37°C for 2 h. Subsequently, the blocking solution was discarded, the plate was placed in a drying room to dry, and stored at 2 to 8 °C for later use.
(2) Dilution of sample: The collected mouse serum was diluted to 1:500 with PBS solution containing 20% NBS (newborn bovine serum) for subsequent quantitative detection.
(3) Denaturation treatment of sample: 15 µL of the above diluted serum sample was taken and thoroughly mixed with 7.5 µL of denaturation buffer (15% SDS, dissolved in 20mM PB7.4), and reacted at 37°C for 1 hour. Subsequently, 90 µL of neutralization buffer (4% CHAPS, dissolved in 20 mM B7.4) was added and mixed thoroughly.
(4) Reaction of sample: 100 µL of the above-mentioned denatured serum sample was added to the reaction plate, and reacted at 37°C for 1 hour. Then the reaction plate was washed 5 times with PBST and spin-dried.
(5) Reaction of enzyme label: 100 µL/well of HBs-A6A7-HRP (Beijing Wantai Biopharmaceutical Co., Ltd.) reaction solution was added to the chemiluminescent plate, and reacted at 37°C for 1 hour. The plate was then washed 5 times with PBST and spin-dried.
(6) Luminescent reaction and measurement: Luminescent liquid (100 µL/well) was added to the chemiluminescent plate, and the light intensity was detected.
(7) Calculation of HBsAg concentration in mouse serum sample: Standard substances were used to conduct parallel experiments, and a standard curve was drawn based on the measurement results of the standard substances. Then, the measured value of light intensity of the mouse serum sample was substituted into the standard curve to calculate the HBsAg concentration in the serum sample to be tested.

### 5.3 Detection of HBsAg in serum of AAV-HBV mice

On the 30th day after the construction of AAV-HBV mouse model, the mouse blood was collected through the retro-orbital venous plexus, and then the change in HBsAg level in the mouse serum was detected. The mice were randomly divided into stratified groups according to HBsAg titer, with 5 mice in each group. 125shIgG1 was injected into mice of HBV-adw serotype, adr serotype, and ayw serotype respectively at a dose of 10 mg/kg by tail vein injection, and a blank control group injected with PBS was set up. Continuous testing was performed for 12 days, and the mouse blood was collected on Day0, Day1, Day4, Day7, and Day11 after treatment through the retro-orbital venous plexus to monitor the HBsAg level in the mouse serum. The results (FIGs. 4A to 4B) showed that compared with the blank control group, 125shIgG1 could maintain HBsAg in the serum concentration range of 100IU/ml for more than 1 week in HBV-adw serotype and HBV-ayw serotype mice, demonstrating a long-lasting therapeutic effect. The above results indicated that 125shIgG1 had broad-spectrum and long-lasting therapeutic effect.

### Example 6: Virus clearance ability of nanobody 125s in HBV transgenic mice

In this example, the virus clearance ability of 125shIgG1 in HBV transgenic mice (gifted from Professor Chen Peizhe of National Taiwan University) was investigated. The HBV transgenic mic were well-known model mice in the art that simulated persistent HBV infection, and were widely used in the field of HBV-related basic research or drug development. The model mice had stable HBV replication level, transcription level and viral protein expression level, and its virological level was comparable to the serum level of patients in the immune clearance phase and HBeAg negative phase. The HBV transgenic mouse model had an intact immune system and immune tolerance to HBV, which was similar to that of HBV-infected individuals to a certain extent. Specifically, 125shIgG1 was injected into HBV transgenic mice via tail vein injection at a dose of 10 mg/kg, with 5 HBV transgenic mice in each group. Continuous testing was performed for 2 weeks, and blood was collected from the mice on Day0, Day2, Day3, Day5, Day7, Day9, Day11, and Day14 after treatment through the retro-orbital venous plexus. HBsAg level in mouse serum was monitored according to the method in Example 5.2. The results (FIG. 5) showed that after the single injection, HBsAg serum concentration in mice maintained in a range of 100IU/ml for 1 week, and HBsAg in the serum of HBV transgenic mice returned to the baseline level after 14 days, which indicated that single injection of 125s was enough to achieve the effect of long-lasting virus clearance.

### Example 7: Identification of the epitope recognized by nanobody 125s

### 7.1 Binding ability of nanobody 125s with denatured HBsAg

Denaturation of HBsAg: 8mmol/L SDS was added to the HBsAg sample, and boiled in a metal bath at 100°C for 10 minutes.

Preparation of reaction plate: The denatured HBsAg and normal HBsAg proteins were diluted with 50mM CB buffer (NaHCO₃/Na₂CO₃ buffer, with final concentration of 50mM, and pH value of 9.6) to a final concentration of 2µg/mL, respectively, and added to a 96-well ELISA plate at 100 µL per well, to perform coating at 2 to 8 °C for 16 to 24 hours, then at 37°C for 2 hours; the plate was washed once with PBST washing solution (20mM PB7.4, 150mM NaCl, 0.1% Tween20), then added with blocking solution (20mM Na₂HPO₄/NaH₂PO₄ buffer solution containing 20% calf serum and 1% casein with a pH value of 7.4) at 200µL per well, and blocked at 37°C for 2 hours; the blocking solution was discarded, and the plate was dried, placed into an aluminum foil bag and stored at 2 to 8 °C for later use.

Elisa detection of reactivity of antibody 125s with denatured HBsAg protein:
125shIgG1 was diluted with 20mM PBS buffer for 3-fold gradient dilution with a starting concentration of 1 µg/mL and a total of 12 gradients. Humanized antibody 162 (which was described in detail in Chinese patent application CN201610879693.5) was used as a positive control. 100 µL of the diluted sample was added to each well of the HBsAg plate and placed in a 37°C incubator for reaction for 60 minutes. The ELISA plate was washed 5 times with PBST washing solution, then 100 µL of HRP-labeled goat anti-human (GAH) IgG reaction solution was added to each well, and placed in a 37°C incubator for 30 minutes. After the enzyme labeling reaction step was completed, the ELISA plate was washed 5 times with PBST washing solution, and 50 µL of TMB chromogenic reagent was added to each well, and placed in a 37°C incubator for reaction for 15 minutes. After the color development reaction step was completed, 50 µL of stop solution was added to each well of the reacted ELISA plate, and the OD450/630 value of each well was detected on a microplate reader.

The results were shown in FIG. 6. The results showed that 125s could bind to normal HBsAg, but not to denatured HBsAg.

### 7.2 Construction of pTTShIgG1-HBsAg clone

The nucleotide sequences of the extracellular segments of different lengths of HBsAg on the surface of HBV subtype B virus were constructed into the pTT5hIgG1 vector, and a series of fusion proteins were constructed. In the fusion proteins, the extracellular segment sequences were linked through the linker (G4S)s to the N-terminal of hIgG1-Fc. The 6 HBsAg extracellular segment gene sequences of different lengths listed in Table 2 were constructed into the pTT5hIgG1 vector, thereby obtaining 6 FC fusion proteins (pTTShIgG1-S1, S2, S3, S4, S5, S6).

**Table 2: HBsAg extracellular segments displayed by pTT5hIgG1**

| Name | Extracellular segment position | SEQ ID NO: |
|---|---|---|
| S1 (Surface) | HBsAg-aa101-aa174 | 16 |
| S2 (N56) | HBsAg-aa101-aa156 | 17 |
| S3 (MHR) | HBsAg-aa118-aa156 | 18 |
| S4 (Cter) | HBsAg-aa157-aa174 | 19 |
| S5 (Loop2-Cter) | HBsAg-aa139-aa174 | 20 |
| S6 (MHR-Cter) | HBsAg-aa118-aa174 | 21 |

### 7.3 Expression and purification of pTT5hIgG1-S fusion protein

293F cells were used to express pTTShIgG1- S1, S2, S3, S4, S5, and S6 fusion proteins. 293F cells with a viability rate higher than 95% were prepared, and 200ml of the cells was taken and inoculated into a 1L cell culture flask at a density of 4×10⁶/mL. 0.6 mg of plasmid was taken, mixed with 1.2 mg of PEI, shaken vigorously for 8 seconds, then allowed to stand for 8 minutes, and the mixture was added to 400 ml of the cells. After 4 hours, 200 mL of Freestyle medium was supplemented and placed in a 5% CO₂ incubator at 37°C for expression for 7 days. The cell supernatant was collected and centrifuged at 10,000 rpm for 30 min. The supernatant was taken and subjected to subsequent purification. The purification method was as described in Example 1.

### 7.4 Evaluation of reactivity of antibody 125s with 6 fusion proteins

In this experiment, E6F6 and 129G1 were used as internal reference antibodies. For E6F6 (recognition epitope located in sa) and 129G1 (recognition epitope located in Se), see Zhang TY et al. Prolonged suppression of HBV in mice by a novel antibody that targets a unique epitope on hepatitis B surface antigen. Gut. 2016 Apr;65(4):658-71. doi: 10.1136/gutjnl-2014-308964.

Preparation of reaction plate: The six fusion proteins of HBsAg extracellular segment were diluted with 50mM CB buffer (NaHCO₃/Na₂CO₃ buffer, with final concentration of 50mM and pH value of 9.6) to a final concentration of 1µg/mL, and added at 100 µL per well to a 96-well ELISA plate to perform coating at 2 to 8 °C for 16 to 24 hours, then at 37°C for 2 hours; the plate was washed once with PBST washing solution (20mM PB7.4, 150mM NaCl, 0.1% Tween20); then 200 µL of blocking solution (20mM Na₂HPO₄/NaH₂PO₄ buffer solution with a pH value of 7.4 containing 20% calf serum and 1% casein) was added to each well, and placed at 37°C for blocking for 2 hours; the blocking solution was discarded, the plate was dried, then placed into an aluminum foil bag and stored at 2 to 8 °C for later use.

Elisa detection of reactivity of antibody 125s with various fusion proteins:
125smIgG2 was diluted to 1ug/ml with 20mM PBS buffer. The internal reference antibodies E6F6 and 129G1 were treated in the same way as 125smIgG2. The Elisa plate coated with pTTShIgG1-S1, S2, S3, S4, S5, S6 fusion proteins was taken, 100 µL of diluted sample was added to each well, and placed in a 37°C incubator for reaction for 60 minutes. The Elisa plate was washed 5 times with PBST washing solution, and 100 µL of HRP-labeled goat anti-mouse IgG reaction solution was added to each well, and placed in a 37°C incubator for 30 minutes. After the enzyme labeling reaction step was completed, the Elisa plate was washed five times with PBST washing solution, and 50 µL of TMB chromogenic reagent was added to each well, and placed in a 37°C incubator for reaction for 15 minutes. After the color development reaction step was completed, 50 µL of stop solution was added to each well of the reacted Elisa plate, and the OD450/630 value of each well was detected on a microplate reader. The reactivity of 125s with pTTShIgG1-S1, S2, S3, S4, S5, S6 fusion proteins was determined based on the reading value after the reaction.

Analysis of epitopes recognized by antibody 125s:
Elisa detection results (FIG. 7B) showed that 125smIgG2 had good reactivity with fusion proteins displaying extracellular segments S1, S4, S5, and S6, but had weak reactivity with fusion proteins displaying extracellular segments S2 and S3. The sequence analysis of these extracellular segments showed that the common feature of the extracellular segments S1, S4, S5, and S6 was that they contained incomplete HBsAg aa157 to 174; and the common feature of the extracellular segments S2 and S3 was that they contained incomplete HBsAg aa118 to 156, but did not contain aa157 to 174. The Elisa results also showed that the reactivity of the fusion proteins pTT5hIgG1-S1 and pTT5hIgG1-S4 with the antibody was higher than that of other fusion proteins, and the reactivity of the fusion proteins pTT5hIgG1-S5 and pTT5hIgG1-S6 with the antibody was higher than that of pTT5hIgG1-S2 and pTT5hIgG1-S3. Therefore, it could be determined that the epitope recognized by 125s was aa157 to 174, which was AFAKYLWEWASVRFSWLS. In addition, since the sequence of S4 was shorter than that of S1, S4 was considered to be a preferred core epitope.

### 7.5 Evaluation of reactivity of antibody 125s with Cter region (aa157 to 174) of different genotypes

Conservation analysis of the Cter region (aa157 to 174) of 10 HBV genotypes (A-J) was shown in FIG. 7C. The red box was aa162-167, which was 100% conserved in different genotypes, suggesting that it could contain the major core epitope of nanobody 125s.

### Example 8: Analysis of sensitivity of antibody 125s to amino acid mutations in extracellular segment S1

### 8.1 Construction of pTT5hIgG1-S1 amino acid single point mutation clone

Since 125s had good binding activity with pTT5hIgG1-S1, an amino acid single point mutation was carried out on pTT5hIgG1-S1, and a total of 14 mutant fusion proteins were prepared. The amino acid sequences of these 14 mutants were set forth in SEQ ID NOs: 22-35.

### 8.2 Expression and purification of pTT5hIgG1-S1 amino acid single point mutation fusion proteins

293F cells were used to express the above single point mutation fusion proteins. The cells were placed in a 5% CO₂ incubator at 37°C for expression for 7 days. The cell supernatant was collected and purified.

### 8.3 Reactivity of 125s with pTT5hIgG1-S1 amino acid single point mutation fusion proteins

### 8.3.1 Preparation of reaction plate

The reaction plate was prepared according to the method in Example 7.3.1, and the coating antigen was pTTShIgG1-S 1 amino acid single point mutation fusion protein.

### 8.3.2 Elisa detection of 125s and pTT5hIgG1-S1 amino acid single point mutation fusion proteins

125smIgG2 was diluted to 1ug/mL with 20mM PBS buffer. The Elisa plate coated with pTT5hIgG1-S1 amino acid single point mutation fusion protein was taken, 100 µL of the diluted sample was added to each well, and placed in a 37°C incubator for reaction for 60 minutes. The Elisa plate was washed five times with PBST washing solution, and 100 µL of HRP-labeled goat anti-mouse IgG reaction solution was added to each well, and placed in a 37°C incubator for reaction for 30 minutes. After completing the enzyme labeling reaction step, the Elisa plate was washed five times with PBST washing solution, and 50 µL of TMB chromogenic reagent was added to each well, and placed in a 37°C incubator for reaction for 15 minutes. After completing the color development reaction step, 50 µL of stop solution was added to each well of the reaction microplate, and the OD450/630 value of each well was detected on a microplate reader. The reactivity of 125s with pTT5hIgG1-S1 amino acid single point mutation fusion protein was determined based on the reading value after the reaction, in which the reading value was normalized to obtain the ratio of "response value of 125s and epitope" to "response value of E6F6 and epitope", and it was known that the reactivity of E6F6 with the displayed epitope was only related to the epitope coating concentration, because the epitope of E6F6 was not on Cter, thus, the mutation of 158-174 would not affect the reactivity of E6F6.

The results were shown in FIG. 7D. When a single point mutation occurred at positions 163-165, the binding activity of 125s to the HBsAg extracellular segment fusion protein was significantly reduced, indicating that it was a core epitope; when a single point mutation occurred at positions 158, 160, and 171, the binding activity of 125s to the HBsAg extracellular segment fusion protein decreased to a certain extent; the mutations at positions 161, 162, 167-170, and 172-174 did not significantly affect the binding activity, indicating that they were not core binding site.

### Example 9: Preparation of humanized antibody of 125s

### 9.1 Humanization of nanobody 125s

The humanization of camelid nanobody 125s was carried out based on the CDR grafting method. By searching the gene database through IMGT, the human germline gene variable region sequence that had the highest homology with the alpaca antibody 125s VHH region was found first. After homology analysis, the germline gene sequence of IGHV3-23*04 was used as the template for engineering humanized antibody heavy chain. At the same time, since the germline gene did not contain the FR4 required for the part to be engineered, the FR4 part should be compared separately, and the IGHJ5*02 germline gene sequence was used as the template for engineering humanized antibody heavy chain FR4. The CDR regions of the alpaca antibody 125s VHH were grafted onto the FR framework regions of the human template. At the same time, the FR regions of the alpaca antibody and the human germline gene were subjected to sequence alignment, and the differential amino acids were selectively subjected to back mutation, and finally one humanized heavy chain was designed to obtain one humanized antibody, named h125s-26, VHH sequence of which was set forth in SEQ ID NO: 13.

### 9.2 Preparation of humanized antibody

The variable region sequence of the humanized antibody was linked to the N-terminal of the human IgG1 Fc fragment (SEQ ID NO: 14) to obtain nanobody-Fc fusion protein h125s-26-hIgG1. Specifically, the variable region sequence of the humanized antibody was constructed into the pTT5-hlgG1 vector (comprising the human Fc region as set forth in SEQ ID NO: 14). In this experiment, the gene synthesis was performed, followed by sequencing analysis (Shanghai Bioengineering Co., Ltd.). The plasmids of gene synthesis were extracted in large quantities using an endotoxin-free plasmid maximal extraction kit (purchased from Beijing Tiangen Biochemical Technology Co., Ltd.). 293F cells were used to express the 125s humanized antibody. The supernatant was taken for subsequent purification, and the purification method was as described in Example 1.3.

### Example 10: ELISA binding activity of 125s humanized antibody to HBsAg

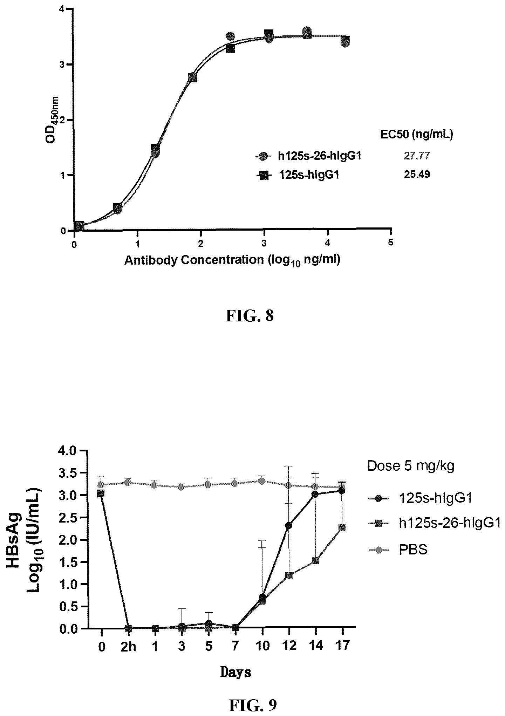

The 125s humanized antibody h125s-26-hIgG1 obtained in Example 9 was taken, and subjected to gradient dilution using SD-1 buffer with a starting concentration of 20 ug/mL and a total of 8 gradients. The ELISA detection method was the same as described in Example 2. The results showed that the EC50 values of the humanized antibody h125s-26-hIgG1 and 125s-hIgG1 for binding HBsAg protein were 27.77ng/mL and 25.49ng/mL, respectively. The results were shown in FIG. 8. The humanized antibody h125s-26-hlgG1 maintained a binding activity to HBsAg comparable to that of 125s-hFc.

### Example 11: Virus clearance ability of 125s humanized antibody in HBV-AAV mice

The h125s-26-hIgG1 and 125s-hIgG1 were separately injected into HBV-AAV mice (HBV-adw serotype) at a single dose of 5 mg/kg via tail vein injection, with 5 HBV transgenic mice in each group. Continuous testing was performed for 17 days, and mouse blood was collected on Day0, 2h, Day1, Day3, Day5, Day7, Day10, Day12, Day14, and Day17 after treatment through the retro-orbital venous plexus. HBsAg levels in mouse serum were monitored according to the method in Example 5.2. The results were shown in FIG. 9. After a single injection, both the h125s-26-hIgG1 and the control antibody 125s-hIgG1 had better HBsAg clearance effects. The two antibodies had similar therapeutic effects in the first 10 days. After 10 days, the HBsAg titer of mice treated with 125s-hFc rebounded rapidly and returned to the baseline level on the 14th day. In contrast, the HBsAg titer of mice treated by the humanized antibody h125s-26 rebounded slowly, and the HBsAg titer had not returned to baseline levels on day 17. It was suggested that the humanized molecule h125s-26 had better antigen clearance ability and long-lasting antigen suppression effect in the AAV/HBV mouse model, and the humanization engineering was successful.

### Example 12: Evaluation of immunogenicity of epitope peptide-Fc fusion protein

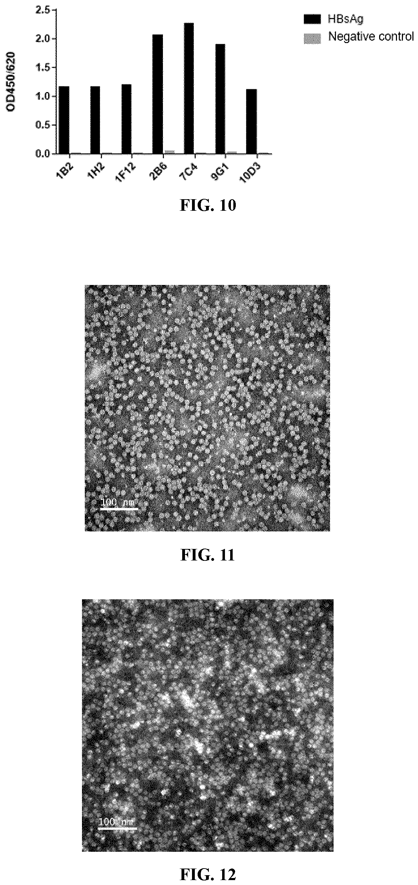

In this example, the immunogenicity of the fusion proteins of the series of HBsAg extracellular segment polypeptides containing S4 (aa157 to aa174) and human IgG1-Fc region in BALB/C mice was investigated. Briefly, recombinant proteins of the above series of polypeptides and human IgG1-Fc region (SEQ ID NO: 14) were prepared respectively, and the recombinant proteins were used to immunize BALB/C mice and produce hybridoma cells. Then the Anti-HBs antibody titers in the secreted supernatants of the hybridoma cells (1B2, 1H2, 1F12, 2B6, 7C4, 9G1, 10D3) were detected. The results were shown in FIG. 10. In the immunotherapy groups using the recombinant proteins, anti-HBsAg antibodies could be detected in the mouse serum samples. It was suggested that the Cter segment of the HBsAg extracellular segment had immunogenicity.

### Example 13: Construction and expression of SpyTag-murine ferritin particle and SpyCatcher-Cter protein

B11-SpyTag-ferritin expression vector was constructed based on the sequence of murine ferritin (NCBI protein name: NP_034369.1), its nucleic acid sequence was codon-optimized for HEK293 host cell, and three G₄S linkers were added between the SpyTag fragment and the ferritin fragment. Similarly, SpyCatcher fragment and Cter fragment (SEQ ID NO: 19) were inserted into the B11 prokaryotic expression vector, and the two fragments were linked via three G₄S linkers. The genes were synthesized by General Biosystems (Anhui) Co., Ltd., and His tag was inserted for purification, and finally the genes B11-SpyTag-murine ferritin-his and B11-SpyCatcher-Cter-his were obtained. The amino acid sequence of SpyTag-murine ferritin was set forth in SEQ ID NO: 49, and the amino acid sequence of SpyCatcher-Cter was set forth in SEQ ID NO: 50.

Expression and purification of B11-SpyTag-murine ferritin:
After the gene was transformed into BL21 competent cells, IPTG was used to induce expression at 37°C. After ultrasonic disruption of the cells, the cell supernatant was collected and heat-treated in a water bath at 70°C for 10 min. After ammonium sulfate precipitation, a gel chromatography column was used for purification, and the sample was stored at 4°C. The transmission electron microscope results (FIG. 11) showed that the purified SpyTag-murine ferritin was a nanoparticle structure with uniform size, stable structure and morphology, and had a diameter of about 20nm.

Expression and purification of B11-SpyCatcher-Cter:
After the gene was transformed into BL21 competent cells, IPTG was used to induce expression at 37°C. After ultrasonic disruption of the bacterial cells, the supernatant was taken and passed through a Ni-Agarose self-packing column, and the SpyCatcher-Cter protein was eluted with imidazole.

### Example 14: Assembly of SpyTag-murine ferritin and SpyCatcher-Cter

The Spy Tag-Ferritin and SpyCatcher-Cter were mixed in a molar ratio of 1:1, and mixed by inverting upside down overnight at 4°C. After mixing SpyTag-ferritin and SC-Cter, filtration in concentration tube and purification on Superose6 Increase gel filtration column were performed, and a high-purity ferritin-Cter nanovaccine was obtained. Transmission electron microscopy results (FIG. 12) showed that the surface of the ferritin-Cter nanoparticle exhibited a protruding structure, which indicated that SpyTag-murine ferritin and SpyCatcher-Cter were successfully assembled, and demonstrated its potential as a vaccine.

Although certain specific embodiments of the present invention have been described in detail above, those skilled in the art will understand that, in light of all teachings that have been disclosed, various modifications and substitutions can be made to the details without departing from the spirit and gist of the present invention, and all these changes are within the protection scope of the present invention. The scope of the present invention is limited only by the appended claims and any equivalents thereof.

## Claims

1. An nanobody or antigen-binding fragment thereof capable of specifically binding to HBsAg, wherein the nanobody or antigen-binding fragments thereof comprise: CDR1 having the sequence as set forth in SEQ ID NO: 1 or a sequence having a substitution, deletion or addition of one or several amino acids (e.g., a substitution, deletion or addition of 1, 2 or 3 amino acids) as compared thereto; CDR2 having the sequence as set forth in SEQ ID NO: 2 or a sequence having a substitution, deletion or addition of one or several amino acids (e.g., a substitution, deletion or addition of 1, 2 or 3 amino acids) as compared thereto; and, CDR3 having the sequence as set forth in SEQ ID NO: 3 or a sequence having a substitution, deletion or addition of one or several amino acids (e.g., a substitution, deletion or addition of 1, 2 or 3 amino acids) as compared thereto.

2. The nanobody or antigen-binding fragment thereof according to claim 1, wherein the nanobody or antigen-binding fragment thereof comprises the sequence as set forth in SEQ ID NO: 8, or a sequence having a sequence identity of at least 80%, at least 85%, at least 90%, at least 91%, or at least 92%, at least 93%, at least 94%, at least 95%, at least 96%, at least 97%, at least 98%, at least 99%, or 100% as compared thereto, or a sequence having a substitution, deletion or addition of one or several amino acids (e.g., a substitution, deletion or addition of 1, 2, 3, 4 or 5 amino acids) as compared thereto.

3. The nanobody or antigen-binding fragment thereof according to claim 1, wherein the nanobody or antigen-binding fragment thereof is humanized;
for example, the nanobody or antigen-binding fragment thereof further comprises a heavy chain framework region of a human immunoglobulin (e.g., a heavy chain framework region contained in the amino acid sequence encoded by a human heavy chain germline antibody gene), and the heavy chain framework region optionally comprises one or more (e.g., 1, 2, 3, 4, 5, 6, 7, 8, 9 or 10) back mutations from human residues to camel residues;
for example, the nanobody or antigen-binding fragment thereof comprises FR1 to FR4 as set forth in SEQ ID NOs: 9-12.

4. The nanobody or antigen-binding fragment thereof according to claim 3, wherein the nanobody or antigen-binding fragment thereof comprises the sequence as set forth in SEQ ID NO: 13, or a sequence having a sequence identity of at least 80%, at least 85%, at least 90%, at least 91%, or at least 92%, at least 93%, at least 94%, at least 95%, at least 96%, at least 97%, at least 98%, at least 99%, or 100% as compared thereto, or a sequence having a substitution, deletion or addition of one or several amino acids (e.g., a substitution, deletion or addition of 1, 2, 3, 4 or 5 amino acids) as compared thereto.

5. A polypeptide construct capable of specifically binding to HBsAg, which comprises the nanobody or antigen-binding fragment thereof according to any one of claims 1 to 4, and an immunoglobulin Fc domain;
for example, the immunoglobulin Fc domain is linked to the N-terminal and/or C-terminal (e.g., C-terminal) of the nanobody or antigen-binding fragment thereof optionally via a peptide linker;
for example, the immunoglobulin Fc domain is an Fc domain of an IgG (e.g., an Fc domain of IgG1, IgG2, IgG3 or IgG4);
for example, the immunoglobulin Fc domain is a human or murine immunoglobulin Fc domain, such as an Fc domain of a human or murine IgG (e.g., an Fc domain of a human or murine IgG1, IgG2, IgG3 or IgG4);
for example, the immunoglobulin Fc domain comprises the sequence as set forth in SEQ ID NO: 14 or 15, or a sequence having a sequence identity of at least 80%, at least 85%, at least 90%, at least 91%, or at least 92%, at least 93%, at least 94%, at least 95%, at least 96%, at least 97%, at least 98%, at least 99%, or 100% as compared thereto, or a sequence having a substitution, deletion or addition of one or several amino acids (e.g., a substitution, deletion or addition of 1, 2, 3, 4 or 5 amino acids) as compared thereto.

6. An isolated nucleic acid molecule, which encodes the nanobody or antigen-binding fragment thereof according to any one of claims 1 to 4, or the polypeptide construct according to claim 5.

7. A vector, which comprises the nucleic acid molecule according to claim 6.

8. A host cell, which comprises the isolated nucleic acid molecule according to claim 6 or the vector according to claim 7.

9. A method for preparing the nanobody or antigen-binding fragment thereof according to any one of claims 1 to 4 or the polypeptide construct according to claim 5, which comprises culturing the host cell according to claim 8 under conditions that allow protein expression, and recovering the nanobody or antigen-binding fragment thereof or the polypeptide construct from a culture of the cultured host cell.

10. A pharmaceutical composition, which comprises the nanobody or antigen-binding fragment thereof according to any one of claims 1 to 4, the polypeptide construct according to claim 5, the isolated nucleic acid molecule according to claim 6, the vector according to claim 7 or the host cell according to claim 8, and a pharmaceutically acceptable carrier and/or excipient.

11. Use of the nanobody or antigen-binding fragment thereof according to any one of claims 1 to 4, the polypeptide construct according to claim 5, the isolated nucleic acid molecule according to claim 6, the vector according to claim 7, the host cell according to claim 8, or the pharmaceutical composition according to claim 10 in the manufacture of a medicament for preventing and/or treating HBV infection or a disease associated with HBV infection (e.g., hepatitis B) in a subject (e.g., a human), for neutralizing the virulence of HBV in vitro or in a subject (e.g., a human), for reducing serum levels of HBV DNA and/or HBsAg in a subject (e.g., a human), and/or for activating a humoral immune response against HBV in a subject (e.g., a subject with chronic HBV infection or a chronic hepatitis B patient).

12. A method, which is used for preventing and/or treating HBV infection or a disease associated with HBV infection (e.g., hepatitis B) in a subject (e.g., a human), for neutralizing the virulence of HBV in vitro or in a subject (e.g., a human), for reducing serum levels of HBV DNA and/or HBsAg in a subject (e.g., a human), and/or for activating a humoral immune response against HBV in a subject (e.g., a subject with chronic HBV infection or a chronic hepatitis B patient), wherein the method comprises: administering to the subject in need thereof an effective amount of the nanobody or antigen-binding fragment thereof according to any one of claims 1 to 4, the polypeptide construct according to claim 5, the isolated nucleic acid molecule according to claim 6, the vector according to claim 7, or the host cell according to claim 8, or the pharmaceutical composition according to claim 10.

13. A conjugate, which comprises the nanobody or antigen-binding fragment thereof according to any one of claims 1 to 4 or the polypeptide construct according to claim 5, and a detectable label;
for example, the detectable label is selected from the group consisting of enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide or biotin.

14. Use of the nanobody or antigen-binding fragment thereof according to any one of claims 1 to 4, or the polypeptide construct according to claim 5, or the conjugate according to claim 13, in the manufacture of a detection reagent for detecting the presence or level of HBsAg protein in a sample, or for diagnosing whether a subject is infected with HBV.

15. An isolated epitope peptide or variant thereof, wherein the epitope peptide or variant thereof comprises an epitope located within amino acid residues 157 to 174 of HBsAg protein, and the epitope comprises at least amino acid residues 163 to 165 of HBsAg protein; the variant differs from the epitope peptide from which it is derived by only a mutation (e.g., a substitution, addition, or deletion) of one or several (e.g., 1, 2, 3, 4, or 5) amino acid residues, and retains the biological function of the epitope peptide from which it is derived;
for example, the epitope comprises at least amino acid residues 163 to 166 of HBsAg protein;
for example, the epitope comprises at least amino acid residues 163 to 165 and amino acid residues 158, 160 and 171 of HBsAg protein;
for example, the epitope comprises at least amino acid residues 163 to 166 and amino acid residues 158, 160 and 171 of HBsAg protein.

16. The epitope peptide or variant thereof according to claim 15, wherein the epitope is a conformational epitope, which consists of at least 3, at least 4, at least 5, at least 6, at least 7, at least 8, at least 9, or at least 10 non-consecutive amino acid residues located within amino acid residues 157 to 174 of HBsAg protein, and comprising at least amino acid residues 163 to 165 of HBsAg protein;
for example, the epitope comprises at least amino acid residues 163 to 166 of HBsAg protein;
for example, the epitope comprises at least amino acid residues 163 to 165 and amino acid residues 158, 160 and 171 of HBsAg protein;
for example, the epitope comprises at least amino acid residues 163 to 166 and amino acid residues 158, 160 and 171 of HBsAg protein.

17. The epitope peptide or variant thereof according to claim 15 or 16, wherein the epitope peptide consists of at least 5 (e.g., at least 6, at least 7, at least 8, at least 9, at least 10, at least 11, at least 12, at least 13, at least 14, at least 15, at least 16, at least 17, at least 18, at least 19, at least 20) and/or at most 80 (e.g., at most 75, at most 70, at most 65, at most 60, at most 55, at most 50, at most 45, at most 40, at most 35, at most 30, at most 25, at most 20) consecutive amino acid residues of HBsAg protein, and comprises the epitope;
for example, the epitope peptide consists of 5 to 80 (e.g., 5 to 75, 5 to 70, 5 to 65, 5 to 60, 5 to 55, 5 to 50, 5 to 45, 5 to 40, 5 to 35, 5 to 30, 5 to 25, 5 to 20; such as 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 74, 75 or 80) consecutive amino acid residues of HBsAg protein, and comprises the epitope.

18. The epitope peptide or variant thereof according to any one of claims 15 to 17, wherein the epitope peptide comprises at least amino acid residues 157 to 174 of HBsAg protein;
for example, the amino acid residues 157 to 174 of HBsAg protein are set forth in any one of SEQ ID NOs: 19 and 38 to 44.

19. The epitope peptide or variant thereof according to any one of claims 15 to 18, wherein the variant does not comprise a mutation at positions corresponding to amino acid positions 163 to 165 of HBsAg protein;
for example, the variant does not contain a mutation at positions corresponding to amino acid positions 163 to 166 of HBsAg protein;
for example, the variant does not contain a mutation at positions corresponding to amino acid positions 162 to 167 of HBsAg protein;
for example, the variant further does not contain a mutation at position corresponding to amino acid position 157 of HBsAg protein;
for example, the variant further does not contain a mutation at position corresponding to amino acid position 171 of HBsAg protein;
for example, the variant further does not contain a mutation at one or more (e.g., 1, 2, or 3) of amino acid positions corresponding to the following positions of HBsAg protein: 158, 160.

20. The epitope peptide or variant thereof according to any one of claims 15 to 19, wherein the variant contains a mutation at one or more (e.g., 1, 2, 3, 4 or 5) of amino acid positions corresponding to the following positions of HBsAg protein: 161, 162, 167, 168, 169, 170, 172, 173, 174;
for example, the mutation is an amino acid substitution, such as a conservative substitution;
for example, the amino acid substitution comprises substitution of the amino acid at the position with alanine.

21. The epitope peptide or variant thereof according to any one of claims 15 to 20, wherein the epitope peptide or variant thereof comprises at least the amino acid sequence of: AX₁X₂X₃X₄X₅WEWAX₆X₇X₈X₉SX₁₀X₁₁X₁₂ (SEQ ID NO: 51); wherein,
X₁ (corresponding to amino acid position 158 of HBsAg protein) is F or L;
X₂ (corresponding to amino acid position 159 of HBsAg protein) is A or G;
X₃ (corresponding to amino acid position 160 of HBsAg protein) is K or R;
X₄ (corresponding to amino acid position 161 of HBsAg protein) is any natural amino acid (e.g., Y, F or A);
X₅ (corresponding to amino acid position 162 of HBsAg protein) is any natural amino acid (e.g., L or A);
X₆ (corresponding to amino acid position 167 of HBsAg protein) is any natural amino acid (e.g., S or A);
X₇ (corresponding to amino acid position 168 of HBsAg protein) is any natural amino acid (e.g., V or A);
X₈ (corresponding to amino acid position 169 of HBsAg protein) is any natural amino acid (e.g., R, H or A);
X₉ (corresponding to amino acid position 170 of HBsAg protein) is any natural amino acid (e.g., F or A);
X₁₀ (corresponding to amino acid position 172 of HBsAg protein) is any natural amino acid (e.g., W or A);
X₁₁ (corresponding to amino acid position 173 of HBsAg protein) is any natural amino acid (e.g., L or A);
X₁₂ (corresponding to amino acid position 174 of HBsAg protein) is any natural amino acid (e.g., S or A);
for example, X₅ is L, X₆ is S; for example, X₉ is F, X₁₀ is W, X₁₁ is L, and X₁₂ is S;
for example, the epitope peptide or variant thereof comprises at least an amino acid sequence selected from the following: the sequence as set forth in any one of SEQ ID NOs: 19 and 38 to 44.

22. The epitope peptide or variant thereof according to any one of claims 15 to 21, wherein the HBsAg protein comprises the sequence as set forth in SEQ ID NO: 36, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto.

23. The epitope peptide or variant thereof according to any one of claims 15 to 22, wherein the epitope peptide or variant thereof comprises or consists of the sequence as set forth in any one of SEQ ID NOs: 16, 19, 22 to 35, 38 to 44.

24. A recombinant protein, which comprises the isolated epitope peptide or variant thereof according to any one of claims 15 to 23, and a carrier protein, and the recombinant protein is not a naturally occurring protein or fragment thereof;
for example, the epitope peptide or variant thereof is linked to a carrier protein optionally via a linker (e.g., a rigid or flexible linker, such as a peptide linker comprising one or more glycines and/or one or more serines).

25. The recombinant protein according to claim 24, wherein the carrier protein is selected from an immunoglobulin Fc domain, such as an IgG Fc domain (e.g., an Fc domain of IgG1, IgG2, IgG3 or IgG4);
for example, the epitope peptide or variant thereof is linked to the N terminal or C terminal (e.g., N terminal) of the immunoglobulin Fc domain optionally via a linker (e.g., a rigid or flexible linker, such as a peptide linker comprising one or more glycines and/or one or more serines);
for example, the immunoglobulin Fc domain comprises the sequence as set forth in SEQ ID NO: 14.

26. The recombinant protein according to claim 24, wherein the carrier protein is selected from a protein capable of self-assembling to form nanoparticle;
for example, the protein capable of self-assembling to form nanoparticle is a ferritin, such as a murine ferritin;
for example, the epitope peptide or variant thereof is linked to the N-terminal or C-terminal (e.g., N-terminal) of the protein capable of self-assembling to form nanoparticle through a peptide bond or an isopeptide bond.

27. The recombinant protein according to claim 26, wherein the epitope peptide or variant thereof is linked to the N-terminal or C-terminal (e.g., the N-terminal) of the protein capable of self-assembling to form nanoparticle through an isopeptide bond;
for example, the isopeptide bond is formed by a protein-protein binding pair;
for example, the protein-protein binding pair consists of a first member (e.g., a first peptide tag) and a second member (e.g., a second peptide tag), wherein the first member and the second member are linked via the isopeptide bond, and, the first member is linked to the N-terminal or C-terminal of the epitope peptide or variant thereof optionally via a first linker (e.g., a rigid or flexible linker, such as a peptide linker comprising one or more glycines and/or one or more serines) to form a first protein, and the second member is linked to the N-terminal or C-terminal (e.g., N-terminal) of the protein capable of self-assembling to form nanoparticle optionally via a second linker (e.g., a rigid or flexible linker, such as a peptide linker containing one or more glycines and/or one or more serines) to form a second protein;
for example, the first protein and/or the second protein may further comprise a protein tag (e.g., at its N-terminal or C-terminal);
for example, the protein-protein binding pair is selected from the group consisting of: (i) SpyTag:SpyCatcher pair, (ii) SpyTag:KTag pair, (iii) Isopeptag:pilin-C pair, (iv) SnoopTag or SnoopTagJr:SnoopCatcher pair, (v) SpyTag002:SpyCatcher002 pair, (vi) RrgATag, RrgATag2 or DogTag:RrgACatcher pair, (vii) IsopepTag N:Pilin N pair, (viii) PsCsTag:PsCsCatcher pair;
for example, the protein-protein binding pair is a SpyTag:SpyCatcher pair; for example, the SpyTag comprises the sequence as set forth in SEQ ID NO: 46; for example, the SpyCatcher comprises the sequence as set forth in SEQ ID NO: 47 or 48.

28. The recombinant protein according to claim 27, which is formed by linking the first protein and the second protein via an isopeptide bond, wherein:
the first protein comprises the epitope peptide or variant thereof and a first member of SpyTag: SpyCatcher pair, and the first member is linked to the epitope peptide or variant thereof (e.g., linked to the N-terminal or C-terminal of the epitope peptide or variant thereof) optionally via a first linker;
the second protein comprises the protein capable of self-assembling to form nanoparticle and a second member of SpyTag: SpyCatcher pair, and the second member is linked to the protein capable of self-assembling to form nanoparticle (e.g., linked to the N-terminal of the protein capable of self-assembling to form nanoparticle) optionally via a second linker;
for example, the first protein comprises the epitope peptide or variant thereof and SpyCatcher or active fragment thereof (e.g., an N-terminal truncation), and the second protein comprises the protein capable of self-assembling to form nanoparticle and SpyTag;
for example, the first protein comprises the sequence as set forth in SEQ ID NO:49, and the second protein comprises the sequence as set forth in SEQ ID NO:50.

29. An isolated nucleic acid molecule, which comprises a nucleotide sequence encoding the epitope peptide or variant thereof according to any one of claims 15 to 23, or the recombinant protein according to any one of claims 24 to 28.

30. A vector, which comprises the isolated nucleic acid molecule according to claim 29.

31. A host cell, which comprises the isolated nucleic acid molecule according to claim 29 or the vector according to claim 30.

32. A method for preparing the epitope peptide or variant thereof according to any one of claims 15 to 23, or the recombinant protein according to any one of claims 24 to 28, which comprises culturing the host according to claim 31 under conditions that allow protein expression, and recovering the epitope peptide or variant thereof or the recombinant protein from a culture of the cultured cell.

33. A multimer comprising multiple monomers, wherein each monomer is independently selected from the recombinant protein according to any one of claims 24 to 28;
for example, the multimer is a dimer, trimer or tetramer;
for example, the monomers are identical to each other;
for example, the monomers are identical to each other and are selected from the recombinant protein according to claim 25.

34. A particle, displaying on its surface the isolated epitope peptide or variant thereof according to any one of claims 15 to 23;
for example, the particle is a nanoparticle, virus-like particle (VLP) or core-like particle (CLP);
for example, the particle comprises or consists of the recombinant protein according to any one of claims 24 to 28 or the multimer according to claim 33.

35. A pharmaceutical composition, which comprises the epitope peptide or variant thereof according to any one of claims 15 to 23, the recombinant protein according to any one of claims 24 to 28, the isolated nucleic acid molecule according to claim 29, the vector according to claim 30, the host cell according to claim 31, the multimer according to claim 33, or the particle according to claim 34;
for example, the pharmaceutical composition is a vaccine, such as a protein vaccine or a nucleic acid vaccine;
for example, the pharmaceutical composition further comprises a pharmaceutically acceptable carrier and/or excipient (e.g., adjuvant).

36. Use of the epitope peptide or variant thereof according to any one of claims 15 to 23, the recombinant protein according to any one of claims 24 to 28, the isolated nucleic acid molecule according to claim 29, the vector according to claim 30, the host cell according to claim 31, the multimer according to claim 33, the particle according to claim 34, or the pharmaceutical composition according to claim 35, in the manufacture of a preparation, the preparation being used for reducing serum levels of HBV DNA and/or HBsAg in a subject (e.g., a human), inducing an immune response (e.g., a humoral immune response) against HBV in a subject (e.g., a human), and/or preventing and/or treating HBV infection or a disease related to HBV infection (e.g., hepatitis B) in a subject (e.g., a human);
for example, the preparation is a vaccine, such as a protein vaccine or a nucleic acid vaccine;
for example, the subject is a subject who is not infected with HBV or a subject who has been infected with HBV, such as a subject with chronic HBV infection or a chronic hepatitis B patient.

37. A method for reducing serum levels of HBV DNA and/or HBsAg in a subject (e.g., a human), inducing an immune response (e.g., a humoral immune response) against HBV in a subject (e.g., a human), and/or preventing and/or treating HBV infection or a disease related to HBV infection (e.g., hepatitis B) in a subject (e.g., a human), wherein the method comprises: administering to the subject in need thereof an effective amount of the epitope peptide or variant thereof according to any one of claims 15 to 23, the recombinant protein according to any one of claims 24 to 28, the isolated nucleic acid molecule according to claim 29, the vector according to claim 30, the host cell according to claim 31, the multimer according to claim 33, the particle according to claim 34, or the pharmaceutical composition according to claim 35.

38. An antibody or antigen-binding fragment thereof, which is capable of specifically binding to the epitope peptide or variant thereof according to any one of claims 15 to 23 or an epitope contained therein, the recombinant protein according to any one of claims 24 to 28, the multimer according to claim 33 or the particle according to claim 34;
for example, the antibody or antigen-binding fragment thereof is selected from the group consisting of Fab, Fab', F(ab')₂, Fd, Fv, dAb, complementarity determining region fragment, single-chain antibody (e.g., scFv), nanobody, humanized antibody, chimeric antibody or bispecific or multispecific antibody;
for example, the antibody or antigen-binding fragment thereof competes with the nanobody or antigen-binding fragment thereof according to any one of claims 1 to 4 for binding to HBsAg protein.
